# EUROPEAN PATENT SPECIFICATION

(11) **EP 3 573 524 B1**
(45) Date of publication and mention of the grant of the patent: **30.08.2023**
(21) Application number: 18743990.6
(22) Date of filing: 24.01.2018
(51) Int. Cl.: A61B 5/24

(54) **ELECTROMYOGRAPHY BASED DRUG DELIVERY**
AUF ELEKTROMYOGRAFIE BASIERENDE ARZNEIMITTELABGABE
ADMINISTRATION DE MÉDICAMENT BASÉE SUR L'ÉLECTROMYOGRAPHIE

(30) Priority: 25.01.2017 US 201762450356 P
(43) Date of publication of application: 04.12.2019
(73) Proprietor: Pretel Inc., Memphis, Tennessee 38120 (US); Young, Roger Charles, Memphis, Tennessee 38120 (US)
(72) Inventor: YOUNG, Roger, Charles, Memphis, TN 38120 (US)
(74) Representative: Marks & Clerk LLP
(86) International application number: PCT/US2018/015054
(87) International publication number: WO 2018/140499

(56) References cited:
- GB-A- 1 547 036
- US-A1- 2002 193 670
- US-A1- 2005 267 376
- US-A1- 2007 233 203
- US-A1- 2012 150 010
- US-A1- 2014 012 190
- US-A1- 2014 012 190
- US-A1- 2014 039 340
- US-A1- 2016 120 435
- US-A1- 2016 120 435

## Description

### CROSS-REFERENCE TO RELATED APPLICATIONS

This application claims priority to U.S. Provisional Patent Application No. 62/450,356 filed on January 25, 2017, which is herein incorporated by reference in its entirety.

### BACKGROUND

Induction of labor is undertaken when the patient is not in labor, but fetal or maternal conditions indicate the need for delivery. Commonly encountered indications can include: rupture of membranes at term, infection, pre-eclampsia, and achieving 41 weeks gestation (or 39 weeks with diabetes), chronic hypertension or advanced maternal age. Rates for labor induction are 23% in the US, or approximately 1 million births annually.

Inductions of labor can use intravenous oxytocin administration. Complications of using too little oxytocin include delay of delivery, excessive use of resources, and increasing maternal fatigue after many hours or days of attempting induction. Complications of using too much oxytocin include uterine tachysystole with associated fetal distress and postpartum hemorrhage. Often the induction requires many hours of slowly increasing rates, followed by cessation of induction because of tachysystole associated with fetal heart rate decelerations, then restarting the induction. Inductions that span several days are not uncommon. Additionally, nursing recommendations are to increase the nurse to patient ratio to 1:2 for labor inductions. This can directly increase costs and shifts valuable resources to these patients.

It is difficult to know exactly how the uterus is responding to an oxytocin infusion using currently available recording methods. Hence, decisions to increase or decrease rates of oxytocin infusion are largely based on an inaccurate and incomplete assessment of the uterine response. There are many detailed protocols for oxytocin infusion, which are generally grouped as low-dose or high dose options. Either option can require enhanced nursing and obstetrician observation and input and can rely heavily on the individual experiences of the providers. US2014/012190 A1 and US2005/267376 A1 show labor monitoring systems with infusion control.

### SUMMARY

The present solution discusses a system configured to evaluate the progress of labor induction by monitoring the uterine response to induction drugs, such as oxytocin and assess the physiological status of the uterus. The uterine response to drugs can vary over the course of labor, but by categorizing the uterine response in real time the system can control an infusion system to deliver patient-specific amounts of drugs to the patient that improves patient outcome while providing cost savings. The system can improve patient outcomes by providing additional information and assistance to healthcare professionals to (automatically) guide the drug infusion rates during induction and augmentation of labor. The system can reduce costs by reducing the total time of labor and reducing the resources spent during labor inductions. The system can also automate the process of labor induction.

According to the invention, a drug infusion system includes a memory device and at least one processor executing an infusion controller. The infusion controller receives a plurality of electrical signals. Each of the plurality of electrical signals can be received from a respective area electrode detecting localized bioelectric signal from a uterus. The infusion controller determines the presence of a first rest period. The rest period does not contain a uterine contraction. The infusion controller identifies within the first rest period of the plurality of electrical signals, an oscillatory signal in at least one of the plurality of electrical signals. The oscillatory signal has an amplitude of between about 20 µV and about 200 µV. The infusion controller can adjust, responsive to identifying the oscillatory signal in at least one of the plurality of electrical signals, an infusion rate of a drug.

In some implementations, the infusion controller can determine, within a second rest period of the plurality of electrical signals, that the plurality of electrical signals do not include an oscillatory signal. The infusion controller can increase the infusion rate of the drug. The infusion controller can identify, within the first rest period of the plurality of electrical signals, the oscillatory signal in a subset of the plurality of electrical signals. The infusion controller can increase the infusion rate of the drug based on the subset of the plurality of electrical signals being below a predetermined threshold.

According to the invention, the infusion controller determines a duration of the oscillatory signal in the subset of the plurality of electrical signals. The infusion controller can increase the infusion rate of the drug based on the duration of the oscillatory signal in the subset of the plurality of electrical signals being below a predetermined time threshold. The infusion controller can identify, within the first rest period of the plurality of electrical signals, the oscillatory signal in a subset of the plurality of electrical signals. The infusion controller decreases the infusion rate of the drug based on the subset of the plurality of electrical signals being above a predetermined threshold.

In some implementations, the infusion controller can identify, within a second rest period of the plurality of electrical signals, the oscillatory signal is present in less than half of the plurality of electrical signals. The infusion controller can maintain the infusion rate of the drug based on the oscillatory signal being present is less than half of the plurality of electrical signals.

The infusion controller can determine a duration of the oscillatory signal in the subset of the plurality of electrical signals. The infusion controller can stop the infusion of the drug based on the duration of the oscillatory signal in the subset of the plurality of electrical signals being above a predetermined time threshold. The predetermined time threshold can be between about 60 seconds and about 120 seconds. The drug pump can include a signal processor configured to band-pass filter the plurality of electrical signals between about 0.2 Hz and about 2 Hz.

In some implementations, the signal processor can be configured to generate a plurality of filtered electrical signals band-pass filtered between about 0.2 Hz and about 2 Hz. The infusion controller can determine the presence of the first rest period based on the plurality of filtered electrical signals not having peak to peak oscillations greater than 200 µV.

According to an aspect of the disclosure, a method delivers a labor inducing drug can include receiving, by an infusion controller, a plurality of electrical signals. Each of the plurality of electrical signals can be received from a respective area electrode detecting localized bioelectrical signal from a uterus. The method can include determining, by the infusion controller, the presence of a first rest period. The rest period does not contain a uterine contraction. The method can include identifying, by the infusion controller and within the first rest period of the plurality of electrical signals, an oscillatory signal in at least one of the plurality of electrical signals. The oscillatory signal can have an amplitude of between about 20 µV and about 200 µV. The method can include adjusting, by the infusion controller and responsive to identifying the oscillatory signal in at least one of the plurality of electrical signals, an infusion rate of a drug.

In some implementations, the method can include determining, within a second rest period of the plurality of electrical signals, that the plurality of electrical signals do not include an oscillatory signal. The method can include increasing the infusion rate of the drug.

The method can include identifying, within the first rest period of the plurality of electrical signals, the oscillatory signal in a subset of the plurality of electrical signals. The method can include increasing the infusion rate of the drug based on the subset of the plurality of electrical signals being below a predetermined threshold.

The method can include determining a duration of the oscillatory signal in the subset of the plurality of electrical signals. The method can include increasing the infusion rate of the drug based on the duration of the oscillatory signal in the subset of the plurality of electrical signals being below a predetermined time threshold.

The method can include identifying, within the first rest period of the plurality of electrical signals, the oscillatory signal in a subset of the plurality of electrical signals. The method can include decreasing the infusion rate of the drug based on the subset of the plurality of electrical signals being above a predetermined threshold.

The method can include identifying, within a second rest period of the plurality of electrical signals, the oscillatory signal is present in less than half of the plurality of electrical signals. The method can include maintaining the infusion rate of the drug based on the oscillatory signal being present is less than half of the plurality of electrical signals.

In some implementations, the method can include determining a duration of the oscillatory signal in the subset of the plurality of electrical signals. The method can include stopping the infusion of the drug based on the duration of the oscillatory signal in the subset of the plurality of electrical signals being above a predetermined time threshold. The predetermined time threshold can be between about 60 seconds and about 120 seconds.

The method can include band-pass filtering the plurality of electrical signals between about 0.2 Hz and about 2 Hz. The method can include determining, by the infusion controller, the presence of the first rest period based on the plurality of filtered electrical signals not having peak to peak oscillations greater than 200 µV.

### BRIEF DESCRIPTION OF THE DRAWINGS

The skilled artisan will understand that the figures, described herein, are for illustration purposes only. It is to be understood that in some instances various aspects of the described implementations may be shown exaggerated or enlarged to facilitate an understanding of the described implementations. In the drawings, like reference characters generally refer to like features, functionally similar and/or structurally similar elements throughout the various drawings. The drawings are not necessarily to scale; emphasis instead being placed upon illustrating the principles of the teachings. The drawings are not intended to limit the scope of the present teachings in any way. The system and method may be better understood from the following illustrative description with reference to the following drawings in which:
FIG. 1A illustrates an example system for measuring bioelectrical signals.
FIG. 1B illustrates a cross-sectional view of an example area electrode for use in the system illustrated in FIG. 1A as a dipole field travels parallel to the skin of a patient.
FIG. 1C illustrates a cross-sectional view of the example area electrode for use in the system illustrated in FIG. 1A as a dipole field travels perpendicular to the skin of a patient.
FIGS. 2A and 2B illustrate a schematic of a top view of example area electrodes for use in the system illustrate in FIG. 1A.
FIG. 3 illustrates an example electrode array for use in the system illustrate in FIG. 1A.
FIG. 4 illustrates a flow chart of an example method 400 for measuring bioelectrical signals using system illustrate in FIG. 1A.
FIGS. 5-14 illustrate example recordings make with the area electrodes described herein.
FIG. 15 illustrates an example electromyography based drug delivery system using the area electrodes described herein.
FIGS. 16A-16C illustrates plots of electromyography signals including high voltage and low voltage oscillatory signals captured with the area electrodes 102 described herein.
FIG. 17 illustrates a plot of intrauterine pressure measured using an intrauterine pressure catheter and a plot of six electromyography channels recorded with the area electrodes described herein.
FIG. 18 illustrates a plot of pressure measured using a tocodynamometer and a plot of five electromyography channels recorded with the area electrodes described herein.
FIG. 19 illustrates a flow diagram of an example method to control drug infusion using the system illustrated in FIG. 15.

### DETAILED DESCRIPTION

The presently disclosed patient matter will now be described more fully. The presently disclosed patient matter can, however, be embodied in different forms and should not be construed as limited to the embodiments set forth herein below and in the accompanying Examples. Rather, these embodiments are provided so that this disclosure will be thorough and complete, and will fully convey the scope of the embodiments to those skilled in the art.

### A. BIOELECTRIC MONITOR SYSTEM

The present disclosure describes systems and method for detecting and recording intra-abdominal function, contractions, fetal cardiac activity, and maternal cardiac activity. Particularly, organ-level uterine function and the contractions resulting from labor are explored, as well as the enhanced ability to measure the uterine contractions resulting from labor in patients considered clinically obese. However, the present patient matter is also applicable to other intra-abdominal "smooth muscle" contractions, e.g., contractions in the bowel, abdominal muscles, uterine contractions with and without maternal ambulation, and contractions of the non-pregnant uterus.

When determining if a patient is in true labor, not only does the contraction frequency and the strength of the contractions of the uterus need to be measured and recorded, but substantially each of the regions of the uterus should contract in synchronicity. The regions are defined by the different contractile units of the uterus. In order to determine if the regions of the uterus are contracting in synchronicity, it must be determined what regions of the uterus are contracting. The present disclosure discusses an area electrode with increased spatial resolution - providing the ability to detect the region of origination of the bioelectrical signals generated by the uterus. True labor can then be determined when a predetermined number of the regions begin to synchronously (as a function of timing, strength, and/or frequency) contract.

Past methods to attempt to determine whether the uterus is contracting involved the use of standard pad electrodes and amplifiers, such as those used in electrocardiography (EKG) in order to measure the bioelectrical signals of an organ (in this case the heart) near or beneath the EKG pads. However, when EKG pads are placed on the abdomen of a patient in order to record contractions in the uterus, the resulting recorded signals are unreliable because the electrodes record the summed electrical activity across multiple regions (e.g., contractile units) of the uterus. Consequently, it is difficult to tell which regions of the uterus generate the measured bioelectrical signals and thus it is difficult to determine if the different regions of the uterus are contracting in synchrony. Also, the signals recorded with the EKG pads are generally low with significant noise interference.

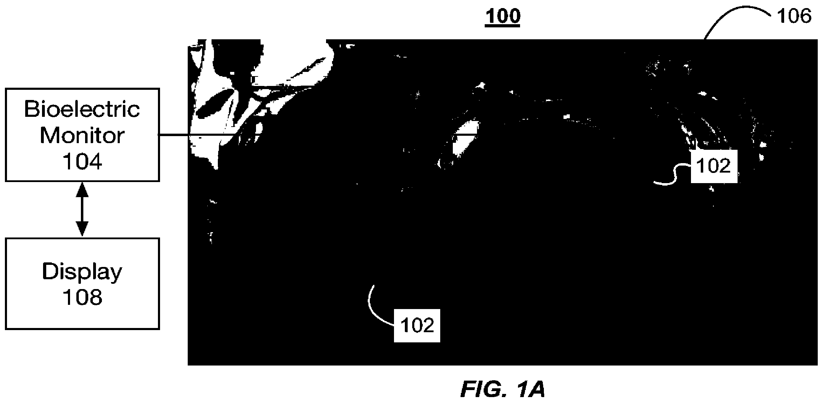

FIG. 1A illustrates an example system 100 for measuring bioelectrical signals. The system 100 includes a plurality of area electrodes 102. Each of the area electrodes 102 are coupled to a bioelectric monitor 104, and the bioelectric monitor 104 uses the area electrodes 102 as inputs to detect electrical signals generated by the patient 106. The signals detected by the bioelectric monitor 104 can be presented to a user via the display 108.

As illustrated, the system 100 can include a bioelectric monitor 104. The bioelectric monitor 104 can be a fetal monitor, electrocardiogram monitor, electromyogram monitor, or other type of patient monitor capable of detecting and/or recording electrical signals from a patient. The bioelectric monitor 104 can be configured to have between 1 and 32 (or more) inputs. The bioelectric monitor 104 can use a signal recorded at one or more of the inputs as a reference or ground signal, which can be subtracted or otherwise removed from the signals recorded at the other inputs of the bioelectric monitor 104. The bioelectric monitor 104 can include an amplifier to amplify the signals received at each of the inputs. The analog signals received from the area electrodes 102 at each of the inputs can be digitized with an analog to digital converter with an 8, 10, 12, 24, or 32-bit resolution. The digitized signals can have a sampling rate between about 128 Hz and about 1 kHz, between about 128 Hz and about 516 Hz, or between about 128 Hz and about 256 Hz. The bioelectric monitor 104 can include the display 108 - for example, the bioelectric monitor 104 and the monitor 108 can be single unit. In other implementations, the display 108 can be a separate device, such as a monitor connected to a desktop computer.

In some implementations, the system 100 is configured to record bioelectric signals originating from the uterus, which can include uterine and fetal bioelectric signals. The uterus includes a plurality of contractile units. Each of the contractile units are about 8 cm x 8 cm, and includes muscle cells grouped into a syncytia. A syncytia is the smallest functional electrical unit in the uterus and is about 1 cm to about 2 cm wide. Each of the syncytia within a contractile unit are coupled together by gap junctions or connexins. Electrical activity does not propagate between separate contractile units of the uterus, but rather the organ-level contractions of the uterus occur through a mechanotransduction recruitment mechanism. Organ-level contractions (e.g., the contraction of substantially all of the contractile units) are indicative of true labor. In other implementations, the system 100 is configured to record additional bioelectric signals such as maternal cardiac signals.

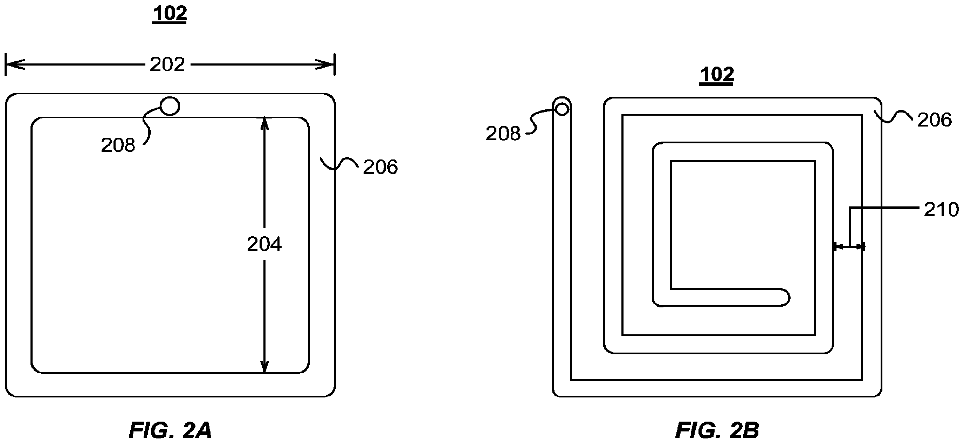

The system 100 can include one or more area electrodes 102. The area electrodes 102 are described in further detail in relation to FIGS. 2-14. As an overview, the area electrodes 102 are ring electrodes that are configured to detect electrical signals originating from the uterus or other muscle-containing intra-abdominal organ. In some implementations, each of the electrodes 102 in the system 100 can be configured the same or differently. For example, each of the area electrodes 102 can have the same shape and size or a different shape and size. The electrodes designed for use in electroencephalogram (EEG) and EKG recordings (e.g., "pad" electrodes) are relatively poor at detecting signals originating from the uterus. The heart has a fixed pacemaker and fixed electrical conduction pathways that coordinate contractions. The largest electrical field vectors generated by the heart are substantially directed parallel with the skin of the patient. However, the uterus generates field vectors that run substantially perpendicular to the skin. The pad-type electrodes used in EEG and EKG recordings fail to detect the field vectors that run substantially perpendicular to the skin. The area electrodes 102 are configured to detect the perpendicular field vectors with a greater signal to noise ratio (SNR) when compared to pad-type electrodes. In some implementations, the area electrodes 102 can be used in combination with the pad electrodes.

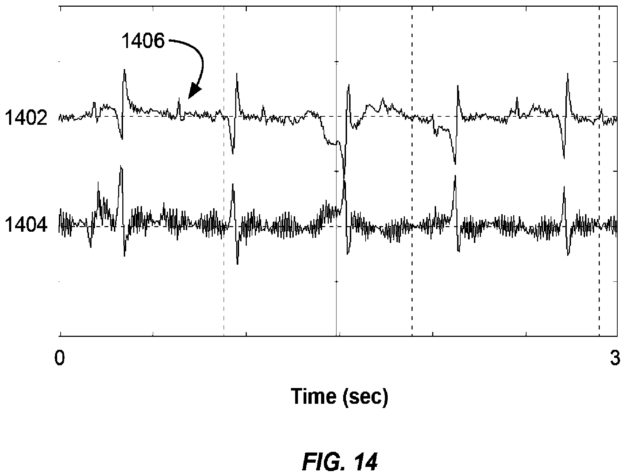

More generally, the area electrodes 102 have an outer diameter (OD) and an inner diameter (ID) with the area there between defining an electrode body. The use of one or more area electrodes 102 placed on the abdomen of the patient can provide larger signals with improved spatial resolution. In order to increase spatial resolution and/or signal strength of bioelectrical signals originating in the uterus with greater accuracy and efficiency, one, two or more area electrodes can be arranged on the abdomen of the patient. In some implementations, the area electrodes 102 are configured to increase signal detection to bioelectric signals arising from a source perpendicular to the area electrode 102 when placed, and to substantially reject signals originating from sources parallel to the area electrode 102. For example, the area electrode 102 can reject about 90%, 80%, 70%, 60%, 50%, 40%, 30%, and 20% of signals originating from sources parallel to the area electrode 102. In some implementations, as also illustrated in reference to FIG. 14 below, the area electrode 102 can be configured to substantially reject other signals that are not of interest. The signals not of interest can include 60 Hz environmental noise, "shot" noise or other noise generated by the movement of the area electrode 102, and biological noise such as the electrical signals generated from muscles not of clinical interest at the time of the recording.

In some implementations, the shape of the area electrode 102 is configured to enable the area electrode 102 to substantially reject electrical signals of non-interest (e.g., noise and those generated substantially parallel to the surface of the skin). For example, electrically active tissue generates an oscillating dipole field. The dipole field is transmitted through the body, where it can be detected by an electrode. When the dipole comes into contact with the electrode, a chemical reaction occurs and releases (or absorbs) electrons and creates a voltage differential that is measured by the bioelectric monitor 104. For a solid, pad style electrode, a dipole field hitting the pad electrode from any direction can create a voltage differential that is measured by the bioelectric monitor 104. In comparison, the area electrode 102 can generate a different magnitude voltage differential responsive to the angle at which the dipole comes into contact with the area electrode 102.

In some implementations, the area electrode 102 includes a conductive gel layer. The gel layer can substantially cover the patient facing portion of a metal layer of the area electrode 102. The gel layer can include electrolytes and can improve electrical coupling between the metal of the area electrodes 102 and the patient's skin by reducing the resistance across the skin-metal barrier.

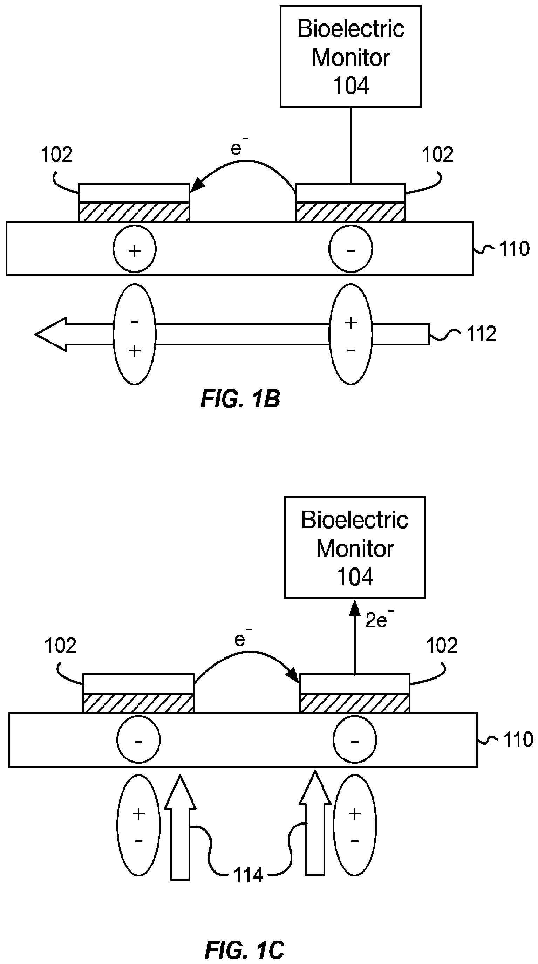

FIG. 1B illustrates a cross-sectional view of an example area electrode 102 as a dipole field travels parallel to the skin. FIG. 1B illustrates two portions of an area electrode 102, which are electrically coupled together via an out of plane portion of the area electrode 102. The area electrode 102 is coupled to the skin 110 as a dipole field 112 travels right to left and parallel to the patient's skin 110, as may occur with a dipole field generated by the maternal heart. The area electrode 102 is coupled to a bioelectric monitor 104. As the dipole field 112 travels right to left and comes into contact with the right portion of the area electrode 102, an electron is released. The electron travels to the left portion of the area electrode 102 where the generated voltage differential generates the reverse chemical reaction than that which occurred in the right portion of the area electrode 102. The electron generated at the right portion of the area electrode 102 is consumed in the chemical reaction occurring at the left portion of the area electrode 102. Because the electron released on the right portion of the area electrode 102 is consumed by the chemical reaction occurring on the left portion of the area electrode 102, the electron does not generate a voltage differential to be detected by the bioelectric monitor 104.

FIG. 1C illustrates a cross-sectional view of the example area electrode 102 as a dipole field 114 travels perpendicular to the skin. As illustrated in FIG. 1C, the dipole field 114 is traveling up from below the area electrode 102. For example, the dipole field may have originated in the uterus or a fetus. The dipole field 114 contacts both portions of the area electrode 102 substantially the same time, which synchronizes the chemical reactions and the release of electrons from both portions of the area electrode 102. The synchronized release of electrons enables the bioelectric monitor 104 to record voltage differential generated by the two electrons.

In some implementations, the system 100 includes a plurality of area electrodes 102. The system 100 can include between about 1 and about 8 area electrodes 102, between about 1 and about 6 area electrodes 102, or between about 1 and about 4 area electrodes 102. In some implementations, the area electrodes 102 can be used in combination with one or more standard pad electrodes. The use of a plurality of area electrodes 102 can enable the detection of the orientation and originating location of bioelectrical signals.

In some implementations, the area electrodes 102 can be placed in various arrangements on the abdomen of the patient. The area electrodes 102 can be arranged in an overlapping or non-overlapping manner. In an overlapping configuration, the area from which the area electrodes 102 detect signals can overlap without the area electrodes 102 physically overlapping. For example, a relatively smaller area electrode 102 may be placed within a relatively larger area electrode 102. When the area electrodes 102 are overlapping, the areas of the electrodes themselves may be slightly different, but the overlapping area electrodes can share, at least, a portion of the same region of, for example, the abdomen. In some implementations, the overlapping area of the area electrodes 102 can be concentric, so that the area electrodes 102 also share a same center point. In some implementations overlapping area electrodes 102 can increase spatial resolution. In some implementations, using non-overlapping area electrodes 102 can increase the signal strength recorded by the area electrodes 102. The electrodes 102 can be referenced to concentrically placed reference electrodes or to remote reference electrodes (e.g., electrodes not within the inner, open area of the electrode 102). In some implementations, for obese patients, signal strength is generally reduced due to fat impeding bioelectric access to intra-abdominal organs. Accordingly, when measuring bioelectric signals from obese patients, the non-overlapping configurations may be used.

FIG. 2A illustrates a schematic of a top view of an example area electrode 102. The area electrode 102 has an outer diameter 202 and an inner diameter 204. The space between the inner diameter 204 and the outer diameter 202 defines the electrode body 206. The area electrode 102 also includes a connector 208.

As illustrated, the area electrode 102 is square shaped with rounded corners. In other implementations, the area electrode 102 is square shaped with blunt corners, rectangular shaped (with or without rounded corners), circular shaped, oval shaped, hexagonal shaped, or any other polygonal shape. The area electrode 102 illustrated in FIG. 2A forms a closed polygonal shape. In other implementations, the area electrode 102 can have an open configuration - for example, a circular shape with an open portion in the circular body of the area electrode 102. In some implementations, the outer diameter 202 is between about 3 cm and about 36 cm, between about 5 cm and about 25 cm, between about 5 cm and about 15 cm, or between about 5 cm and about 10 cm. The inner diameter can be between about 3 cm and about 30 cm, between about 4 cm and about 24 cm, between about 4 cm and about 14 cm, or between about 4 cm and about 9 cm. In some implementations, the width of the electrode body (e.g., the difference between the outer diameter 202 and the inner diameter 204) is between about 0.5 cm and about 5 cm, between about 0.5 cm and about 2.5 cm, between about 0.5 cm and about 1.5 cm, or about 0.5 cm and about 1.2 cm wide. In some implementations, the total area of the electrode body 206 is between about 5 cm² and about 900 cm², about 50 cm² and about 800 cm², about 100 cm² and about 600 cm², or about 200 cm² and about 400 cm². In some implementations, the electrode body 206 is formed from a metal layer. The metal layer can include silver-chloride, graphite, or other metal used for medical grade electrodes.

In some implementations, the size of the outer diameter 202, inner diameter 204, and electrode body 206 is configured responsive to the anatomical features generating the bioelectric signals. In some implementations, the size of the area electrode 102 is designed to be about the size of the largest contractile unit generating bioelectric signals. For example, when the area electrode 102 is configured to measure bioelectric signals from the uterus, which has a contractile unit of about 8 cm x 8 cm, the outer diameter 202 of the area electrode 102 may be set to about 8 cm. In some implementations, at least one feature of the area electrode 102 is configured to be smaller than the tissue-level dipole oscillator. For the uterus, the tissue-level dipole oscillator is the syncytia, which is about 2 cm x 2 cm. Accordingly, when the area electrode 102 is configured to measure bioelectric signals from the uterus the electrode body 206 has a width (e.g., the distance from the inner diameter 204 to the outer diameter 202) of about 1.5 cm.

In some implementations, the size and shape of the area electrode 102 enable the area electrode 102 to act like an antenna and resonate with the oscillating bioelectric signals. The frequency (f), propagation speed (c), and wavelength (λ) of the moving dipole field that generates the bioelectric signals are related by the wave equation, where λ = c/f. In some implementations, the bioelectric signal generated during uterus contractions has a frequency about 1 Hz. The rate of propagation of the bioelectric signal is about 3 cm/sec. Using the wave equation, the wavelength of the signal is about 3 cm. In some implementations, an antenna couples to a signal when one of the dimensions of the antenna is between 1/4 and 1/2 of the signal wavelength. In this example, an antenna should have at least one dimension between about 0.75 cm and about 1.5 cm. Accordingly, in this example where the area electrode 102 is configured to detect contractions from a contractive unit of the uterus, the outer diameter of the area electrode 102 can be about 8 cm (corresponding to the size of a contractile unit) and the width of the electrode body can be about 1.5 cm (1/2 of the signal wavelength) to enable the area electrode 102 to couple to the bioelectrical signal generated by the contractive unit.

In some implementations, the size and shape of the area electrode 102 is selected responsive to the size, weight, and body mass index (BMI) of the patient. In some implementations, obese patients may require relatively larger area electrodes 102, such that an area electrode can be dimensioned specifically for a larger patient. For example, the area electrode 102 used for an obese patient may have a rounded square shape with outer sides measuring at 21 cm long and inner sides measuring at 18 cm long. In comparison, the area electrode 102 for a non-obese patient may have outer sides measuring about 9 cm and inner sides measuring about 6 cm.

In some implementations, obese patients require special consideration, depending on the degree of obesity. Because area electrodes detect signals from a direction pointed into the patient's abdomen and efficiently gather electrical signals, the number and sizes of the area electrodes used can be customized to optimize electrical signal detection from mildly obese (200 lbs. or a BMI between about 25 and about 30) to massively obese pregnant patients (more than 500 lbs. or a BMI above 30). In massively obese patients, the uterus is often a great distance from the abdominal surface, and electrical signals are attenuated by the intervening fat. At some degree of obesity, signal strength is anticipated to diminish and disperse such that increasing the size of the area electrode will be required to gather enough signal to determine the presence of a contraction. For massive obesity, the size of the electrode may need to be increased such that only very large area electrodes can detect electrical signal. This effect can reduce the optimal number of area electrodes, possibly to as low as one.

Still referring to FIG. 2A, the connector 208 of the area electrode 102 can be an electrical connector that enables the area electrode 102 to be coupled to an input of the bioelectric monitor 104. In some implementations, the connector 208 is a snap or button style connector, similar to those used in EKG electrodes. The snap connectors can replace traditional braided electrode connectors, although a combination of braids and snaps can be used. In some implementations, the snap connectors can be adapted for placement on the boundary of the area electrode, such that the size of the snap connectors can be smaller than the boundary or border width of the area electrodes.

FIG. 2B illustrates an example area electrode 102 configured in a spiral shape. The area electrode 102 has a width between about 3 cm and about 36 cm, between about 5 cm and about 25 cm, between about 5 cm and about 15 cm, or between about 5 cm and about 10 cm. In some implementations, the width of the electrode body (e.g., the difference between the outer diameter 202 and the inner diameter 204) is between about 0.5 cm and about 5 cm, between about 0.5 cm and about 2.5 cm, between about 0.5 cm and about 1.5 cm, or about 0.5 cm and about 1.2 cm wide. In some implementations, the total area of the electrode body 206 is between about 5 cm² and about 900 cm², about 50 cm² and about 800 cm², about 100 cm² and about 600 cm², or about 200 cm² and about 400 cm². In some implementations, the spacing 210 between each ring of the area electrode 102 is about 1/4 of the wavelength (using the above wave equation) of the bioelectric signal to be detected.

In some implementations, the area electrode 102 can also include a plurality of separate electrode bodies. Each of the electrode bodies can be electrically coupled together by, for example, an electrical tether or wire. Each of the separate electrode bodies can be coupled to the patient and spaced apart from one another according to the above-described dimensions. For example, each of the electrode bodies can be a separate pad electrode or a strip electrode. The area electrode 102 may include four separate strip electrodes that are electrically coupled together. Each of the strip electrodes may be 1.5 cm wide and about 4 cm long. The four strip electrodes may be configured in a square shape (e.g., a first pair of the electrodes are placed parallel to one another and about 5 cm apart and a second pair of the electrodes are placed parallel to one another about 5 cm apart and perpendicular to the first pair of electrodes).

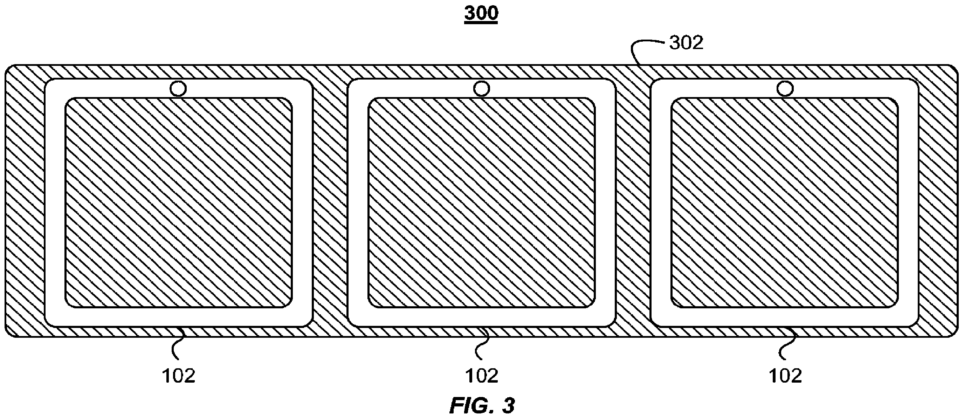

FIG. 3 illustrates an example electrode array 300. The electrode array 300 includes three area electrodes 102 coupled to an adhesive backing 302. In some implementations, the adhesive backing 302 holds each of the area electrodes 102 of the electrode array 300 in a predetermined spatial relationship (e.g., maintains a predetermined spacing between each of the area electrodes 102). In some implementations, the adhesive backing 302 is a repositionable backing that enables the electrode array 300 to be removed and repositioned on a patient.

In some implementations, as illustrated in FIG. 3, each of the electrodes have the same configuration. In other implementations, one or more of the area electrodes 102 can have a different electrode configuration. For example, one of the area electrodes 102 may have a larger inner and outer diameter than the other area electrodes 102, or one or more of the area electrodes 102 can have a different shape. For example, the array can include a larger area electrode 102 and then a smaller area electrode 102 positioned within the larger area electrode 102. In another example, one or more of the area electrodes 102 may be square shaped with rounded corners and one or more of the area electrodes 102 may be rectangular with rounded corners.

As illustrated in FIG. 3, the area electrodes 102 of the array 300 are arranged linearly. In other implementations, the area electrodes 102 are arranged in a nonlinear configuration. For example, the area electrodes 102 may be arranged inside of one another, in a triangular, cross, or circular configuration. The arrangement of the area electrodes 102 on the adhesive backing 302 can improve proper alignment of the area electrodes 102 on the patient. For example, the area electrodes 102 can be configured on the adhesive backing 302 to have a predetermined spacing between each of the area electrodes 102 such that when the electrode array 300 is placed, the proper spacing between neighboring area electrodes 102 is maintained. In some implementations, the electrode array 300 includes between 2 and 12 electrodes, between 2 and 10 electrodes, or between 4 and 6 electrodes.

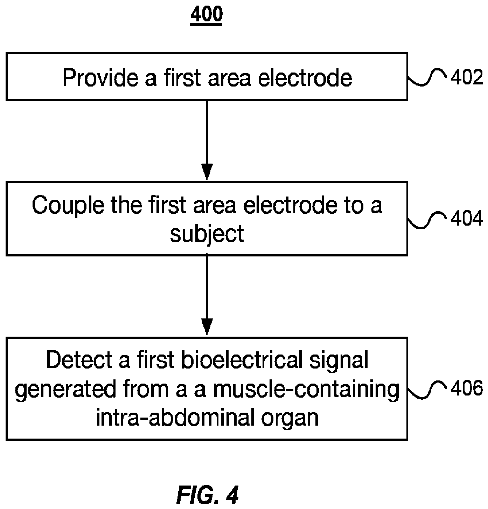

FIG. 4 illustrates a flow chart of an example method 400 for measuring bioelectrical signals. The method 400 can include providing a first area electrode (step 402). The first area electrode is coupled to the abdomen of the patient (step 404). A first bioelectrical signal is detected with the first area electrode from a muscle-containing intra-abdominal organ in a patient (step 406).

As set forth above, the method 400 can include providing a first area electrode (step 402). The first area electrode can be any of the electrodes described herein. For example, the area electrode can be a ring (or other shaped) electrode that includes an electrode body defined in a metal layer by an inner and outer diameter. In some implementations, a plurality of area electrodes is provided. Each of the electrodes may be independent from one another or coupled together. For example, the electrodes may be configured in an electrode array where each of the area electrodes are coupled together by an adhesive layer. The area electrode can have one of a circular shape, a square with rounded corners shape, or a rectangle with rounded corners shape. In some implementations, the area electrodes are configured to detect electrical signals originating substantially perpendicular to a surface of the electrode body, and to substantially reject electrical signals originating substantially lateral to the surface of the electrode body.

Next, the first area electrode is coupled to the patient (step 404). The area electrode can be coupled to the abdomen of the patient. In some implementations, the patient is pregnant and the area electrode is used to detect bioelectrical signals originating from the uterus or a fetus within the uterus. In some implementations, the bioelectrical signals are recorded form the patient to determine if the patient is in labor or to monitor the fetus's heart rate. In some implementations, the area electrodes can include a tacky side that enables the first electrode to be reversibly coupled to the patient. In some implementations, one or more area electrodes can also be coupled to portions of the patient that are remote to the regions of interest (e.g., the uterus). The remotely placed area electrodes 102 can be used as a reference electrode, and can be, for example, coupled to the upper thigh of the patient.

Once the first area electrode is coupled to the patient, a bioelectric signal is recorded with the first area electrode (step 406). The first area electrode can detect bioelectrical signals generated substantially perpendicular to a face of the area electrode coupled to the patient while rejecting bioelectrical signals generated substantially parallel to the face of the area electrode coupled to the patient. In some implementations, the area electrode is configured to detect the bioelectric signals generated from a contractile unit within the uterus. Additional area electrodes may also be coupled to the patient's abdomen to detect bioelectric signals generated from other contractile units with the uterus. If a synchronization pattern is detected between the multiple area electrodes, for example the different area electrodes each detect bioelectric signals indicative of a contraction from the different contractile units, then the system coupled to the area electrodes may determine that the patient is in labor. The bioelectric signal can also be generated from a fetal or a maternal heart.

### B. EXAMPLES

When observed, bipolar electromyography (EMG) recordings using standard pad electrodes have been shown to correlate with uterine contractions. Importantly, in many instances, contractions occur, even though pad-type EMG recordings are silent. In the data reported herein using area electrodes, all patients were in labor, experiencing regular uterine contractions, and the EMG recordings displayed in all figures correlate with a uterine contraction either as reported by the patient, or confirmed using clinical recording of uterine contractions, such as tocodynamometer "toco" recordings or intrauterine pressure catheter (IUPC) recordings. Toco recordings are made from a plunger device that records shape changes of the uterus when contractions occur, and do not rely on the bioelectrical properties of the uterus. For obese patients, toco recording is often unreliable since the shape changes of the uterus are physically distant from the plunger of the toco.

The following Examples have been included to provide guidance to one of ordinary skill in the art for practicing representative embodiments of the presently disclosed patient matter. In light of the present disclosure and the general level of skill in the art, those of skill can appreciate that the following Examples are intended to be exemplary only and that numerous changes, modifications, and alterations can be employed without departing from the scope of the presently disclosed patient matter.

### EXAMPLE 1: COMPARISON OF AREA ELECTRODES TO STANDARD BIPOLAR TECHNIQUE IN ASSESSING REGIONAL CONTRACTIONS

At least one area electrode can be placed onto the abdomen of a patient in order to assess contractions of intra-abdominal muscles. For example, two or more area electrodes can be used to provide a complete assessment of a plurality of contractions in the uterus of the patient. In some aspects, there can be two or more area electrodes or only one area electrode.

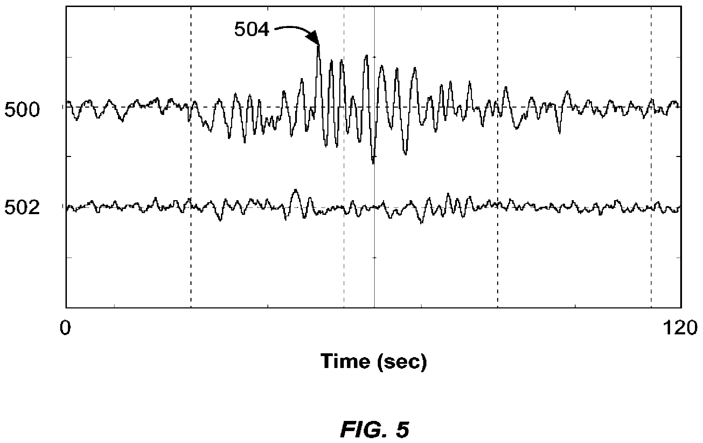

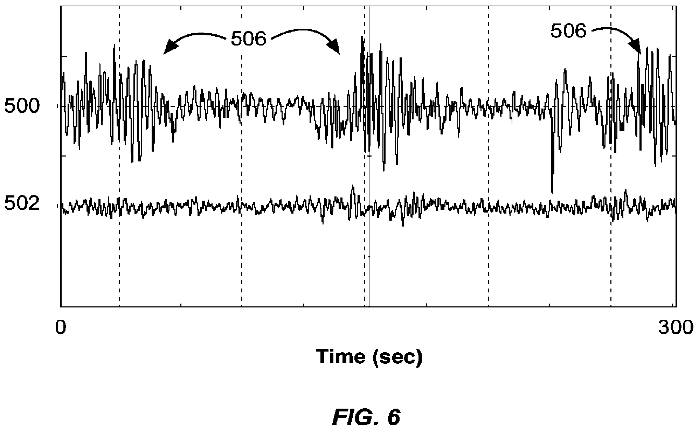

In FIGS. 5 and 6, for example, an electrode as described herein is used to assess regional contractions. Here, an area electrode can be a 12 cm (OD) ring electrode placed on the abdomen in order to assess regional contractions; although other sizes and shapes of the at least one area electrode are contemplated. Using ring electrodes can be beneficial in identifying regional contractions since the ring shape of the area electrode allows separating out different regions.

FIGS. 5 and 6 illustrate two traces of signals recorded from the abdomen of a patient in order to assess regional contractions in intra-abdominal muscles (e.g., the uterus) using a single area electrode of the present disclosure in comparison with a standard bipolar electrode pad technique, i.e., two electrode pads spaced apart. The trace 500 was collected using the above-described ring area electrode of the present disclosure. The area electrode is placed on the abdomen in order to assess regional contractions. The 502 trace was collected using a standard bipolar electrode pad technique.

As illustrated, the trace 502 recorded by the standard bipolar electrode pads is barely discernable due to substantial noise interference, while the trace 500 recorded by the electrode of the present disclosure is clearly discernable. Contractions reported by the patient and as seen on the clinical toco corresponded to the occurrences of the bioelectrical signals of the area electrodes. For example, specifically looking at the signal surrounding the 120 second mark (total time interval being between 3:07:00 and 3:08:00) in the trace 500, a bioelectrical burst 504 is apparent, which is indicative of a contraction of the intra-abdominal muscle. In contrast, the bioelectrical burst of the trace 502 was not visually apparent using standard bipolar electrode pads. FIG. 6 illustrates the same data illustrated in FIG. 5, but with a different time frame. In FIG. 6, it is apparent that three consecutive contractions 506 occurred during the longer time frame, which was detected in the trace 500, but not in the trace 502. In these examples and others, we observe the signal-to-noise ratio of the area electrodes is much superior to pad electrodes.

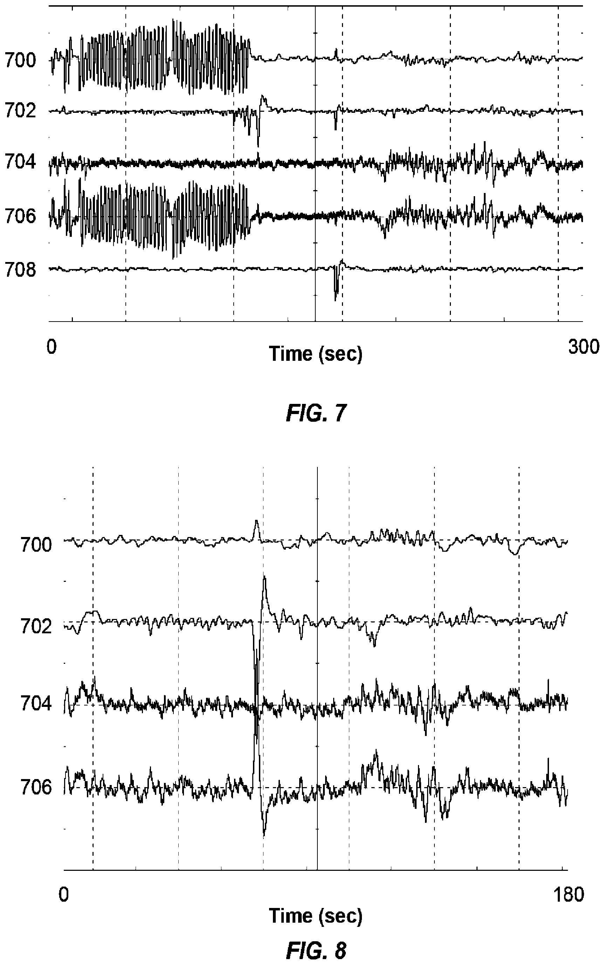

In comparison, FIGS. 7 and 8 illustrate traces generated using two electrodes of the present disclosure (e.g., first and second area electrodes). FIG. 8 illustrates a magnified portion of the traces illustrated in FIG. 7. The first and second area electrodes are placed on the abdomen in order to assess regional contractions. Each of the first and the second area electrodes are 12 cm (OD) ring electrodes as described above. A circle pad electrode is placed in a center of each of the first and the second area electrodes. The first and the second area electrodes can be referenced to one another and to the circle pad electrode placed in the center of the respective first and second electrodes. Referencing can be achieved by subtracting the voltages of one area electrodes from the other in order to determine the total separation of the bioelectrical signals in each of the area electrodes.

In some aspects, the recorded signals from either one or both of the first and second area electrodes and circle pads electrodes can be filtered to reduce the SNR. This can allow any bioelectrical bursts recorded to be more visually discernable. For example, a high pass and/or a low pass filter can be implemented.

In FIG. 7, the top trace 700 is illustrative of the first area electrode referenced to a circular pad electrode in a center of the abdominal ring. The second trace 702 is illustrative of the second area electrode referenced to a pad electrode in a center of the second area electrode. The third trace 704 is illustrative of a pad electrode placed in the center of the second area electrode referenced to a pad electrode placed in the center of the first area electrode. The fourth trace 706 is illustrative of the second area electrode referenced to the center of the first area electrode. The fifth trace 708 is a dual pad arrangement using a bipolar amplifier that is the most commonly used method of recording uterine EMG by others, and is displayed to allow comparison with our method. Each of the top four traces illustrate the voltage differences between electrode pairs of the area electrodes. Here we show that the area electrodes express a directional signaling dependent on the location of the reference electrode, and also dependent on whether the comparison electrode is an area electrode of the present disclosure or a pad.

To summarize the data, trace 700 illustrates a large signal with a specific fingerprint in the first third of the frame, with no signal later. Trace 702 shows only a brief signal occurring at the conclusion of the first third. Trace 704 shows a small voltage signal occurring at the last third of the frame (this signal is easily distinguished, but is not much more than the noise level). Trace 706 shows the same fingerprint signal at the same time as in trace 700, except inverted, plus the same smaller signal seen in trace 704. Trace 708, the bipolar signal, shows only a brief noise in the latter half of the tracing.

Analysis now shows that the large signal seen in trace 700 arises from the area below that first area electrode, as the comparison point is from within that area. This indicates that a contraction is occurring deep in the patient's abdomen, directly beneath the first area electrode. Trace 702 displays voltages arising only from the area beneath the second area electrode, since the comparison point is within this area - and the lack of signal indicates no contraction occurs directly beneath the second area electrode. Trace 704 mimics a pair of bipolar leads probing the signal that occurs on the skin between the area electrodes, and indeed the small signal is observed, but the large signal is completely missed. In trace 706 the second area electrode is referenced to the circle pad electrode remotely located within the first area electrode, and records both the deep and surface signals, but since the signals are referenced to a remote pad, the noise is higher. Thus the area electrode/remote pad combination records both surface and deep electrical signals, but the addition of the surface signals is obtained in a trade-off for increased noise.

Contrastingly, the signal recorded by standard bipolar electrode pads (e.g., trace 708) illustrates that the standard bipolar electrode pads, by themselves, were not able to accurately assess whether a true labor contraction occurred.

To summarize, these traces indicate that (1) area electrodes, as described herein, yield larger electrical signals with less noise, greatly improving SNR over conventional pad electrodes; (2) area electrodes as described herein observe uterine signals when standard pad electrodes do not, suggesting area electrodes probe in a direction pointed into the body, while standard pad probe electrodes measure bioelectrical signals directed parallel with the skin surface; (3) by directly comparing the signals recorded from different pairs of area electrodes, or area/pad pairs, it is possible to assign specific areas of muscle contraction activity within the abdominal cavity; and (4) area electrodes enable a determination to be made of whether a true labor contraction occurred. This directly indicates that area electrodes can delineate the location of the source of the electrical activity within the abdominal cavity, and demonstrates what we have termed spatial resolution, or directionality.

### EXAMPLE 2: COMPARISON OF AREA ELECTRODES TO STANDARD BIPOLAR TECHNIQUE IN ASSESSING REGIONAL CONTRACTIONS IN OBESE PATIENTS

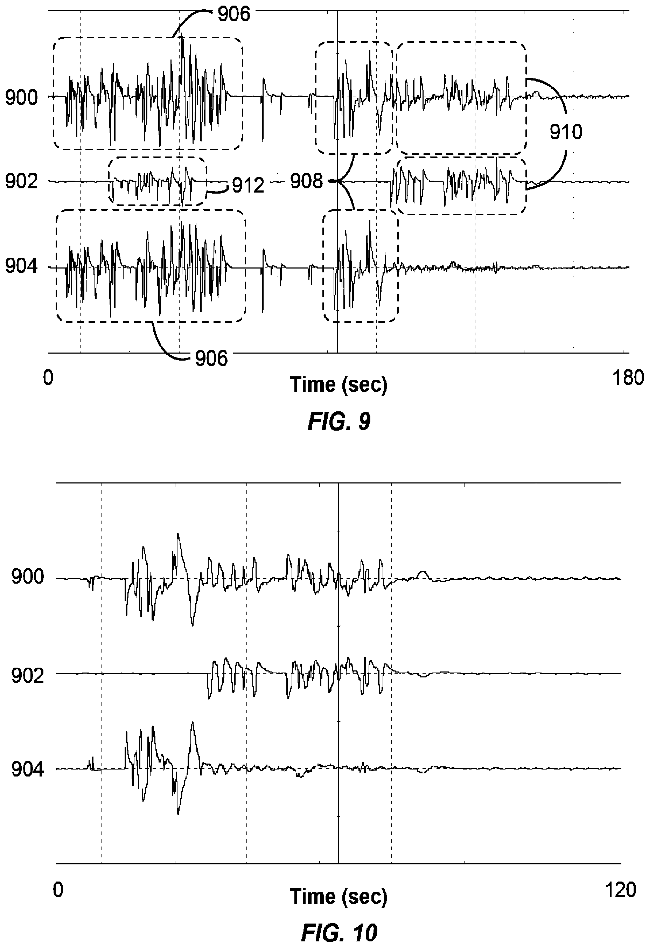

The traces illustrated in FIGS. 9-11 differs from the data illustrated in FIG. 5 because the patient is considered clinically obese, with a body mass index (BMI) of 38 and above. In some aspects, obesity determination can be based on BMI, among other factors. In other aspects, obesity can be based on an abdominal girth measurement, among other factors. Regardless, with patients considered clinically obese, it is much harder to use standard bipolar electrode techniques to assess regional contractions in smooth muscles, like the uterus because the increased body fat impedes access to the smooth muscles. However, area electrodes have been found to record signals better than standard bipolar electrode techniques, as illustrated in Example 1.

In FIGS. 9-11, an analysis of area electrodes is used to assess regional contractions. Specifically, an analysis of area electrode as described herein is illustrated, where each area electrode is formed as a 12 cm (OD) circular ring. However, other shapes and/or sizes of the area electrodes can also be used in assessing regional contractions in obese patients (e.g., a 21 cm x 21 cm rounded square).

The area electrodes were placed on the abdomen of the patient in order to measure bioelectrical bursts in the uterus, where arrangement of the area electrodes can vary. Three area electrodes were arranged in a triangle on the patient's abdomen, with each area electrode having a pad electrode positioned within the area electrode spanned by the area electrode. The area electrode can be numbered in any manner that can allow easy and comprehendible reference to each area electrode. For example, in the arrangement provided by FIGS. 9-11, the area electrodes can be arranged in a triangle and can be numbered 2, 4, 6, respectively, while the pad electrodes within each area electrode can be numbered 1, 3, 5, respectively.

In order to measure the bioelectrical bursts, each of the area electrodes was referenced to one another in order to reflect the voltages in each area electrode. For example, in FIG. 9, the top trace 900 is area electrode 2 minus area electrode 4; the middle trace 902 is area electrode 4 minus area electrode 6; the bottom trace 904 is area electrode 2 minus area electrode 6. As illustrated, the traces are large, approaching 400 µV.

It is seen that there are four time intervals showing electrical activity. The first interval is interval 906 (60 sec total duration) and appears in the trace 900 and 904. The second time interval 908 (10 sec total duration) appears in the trace 900 and 904. The third interval 910 (25 sec total duration) occurs in the traces 900 and 902. Lastly there is a fourth, smaller interval 912 (25 sec total duration), seen best in the middle trace 902.

Remembering that the traces 900, 902, and 904 represent signals from pairs of area electrodes, it is straight forward to assign each signal to a specific area electrode. Beginning with the inverted third interval 910, this is seen in the trace 900 and 902 but not in the trace 904. Thus this signal is from area electrode 4. The inversion reflects 4 being subtracted in one tracing, but not the other.

Next, consider the first interval 906, which is seen in trace 900 and trace 904, without inversion. Hence this signal arises from area electrode 2. Similarly, the second interval 908 is seen in the traces 900 and 904 without inversion and also can be assigned to area electrode 2. Finally, the fourth interval 912 is seen best in the trace 902, indicating the source is either 4 or 6, but the top and bottom tracings partially obscure visually seeing this smaller signal. Detailed analysis reveals, however, that the signal is present inverted in the trace 900, indicating the origin is area electrode 4.

We can therefore conclude that regional contractions beneath each area electrode occur over those time intervals. Furthermore, area electrode 4 demonstrates a regional contraction that occurs at the same time as another regional contraction (e.g., the fourth time interval 912 is simultaneous with the first time interval 906 of area electrode 2) and following another regional contraction (e.g., third time interval 910 follows the second time interval 908 of area electrode 2).

This demonstrates that regional contractions of the uterus can be detected using only area electrodes, and synchronous and asynchronous contractions of specific regions can be detected.

FIG. 10 illustrates an expansion of the time frame near the end of FIG. 9 to allow assessment of the small signals in the trace 904 that occurred after the second time interval 908. The trace 904 has been inverted in FIG. 10 subtracting 6-2 rather than 2-6 as was done in FIG. 9. It was not obvious in FIG. 9 if this small signal was unique, or part of the third interval 910 seen clearly in the tracing 902. Because of this expansion, the phase of oscillations (fingerprint) can now be seen to be unique, indicating the signal is not arising specifically beneath a single area electrode. This indicates that area electrodes 2 and 6 are functioning similarly to pad electrodes, and display a signal that originated along the skin between the area electrodes. To emphasize the scale of the tracings, these "small signals" are still 50 µV, and quite sufficient to define contractile activity. Thus, area electrodes separated by some distance retain all the advantages of area electrodes with regard to gathering large signals and reporting directionality, but furthermore can report surface signals similarly to pad electrodes. This dual functioning can assist with determining if the bioelectrical signal arising from the entire uterus is captured by a specific physical arrangement of the area electrodes (in this example it is not between area electrode 2 and 6). Thus, the presence or absence of surface signals will assist with defining the fidelity of capturing all electrical signals originating from the entire intra-abdominal cavity.

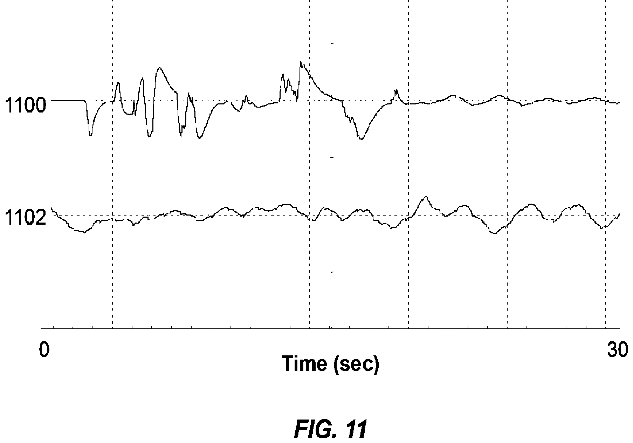

In comparison with both FIGS. 9 and 10, FIG. 11 illustrates the same data expanded only about area electrode 2 referenced to area electrode 6. The first trace 1100 illustrates area electrode 2 minus area electrode 6. The second trace 1102 illustrates the pad electrode 1 in the center of area electrode 2 referenced to the pad electrode 5 in the center of the area electrode 6 (electrode 1 - electrode 5). From this data, the pad electrodes reveal a barely visible maternal heart rate signal, but no clear uterine bioelectrical signal. A high pass filter of 0.4 Hz and a low pass filter of 0.8 Hz were used to filter the traces illustrated in FIG. 11. Using the pads in the centers of the area electrode ensured that the pads spanned an area with known contractile activity. These data indicate that the standard pad electrodes, in some cases, do not display signals, despite the proven occurrence of contractions underneath the electrodes. This lends further support to our other observations that area electrodes record signals that originate from within the patient's abdomen, and the pad electrodes record voltage differences across the surface of the skin.

### EXAMPLE 3: COMPARISON OF AREA ELECTRODES TO STANDARD BIPOLAR TECHNIQUE IN ASSESSING ACTIVE LABOR

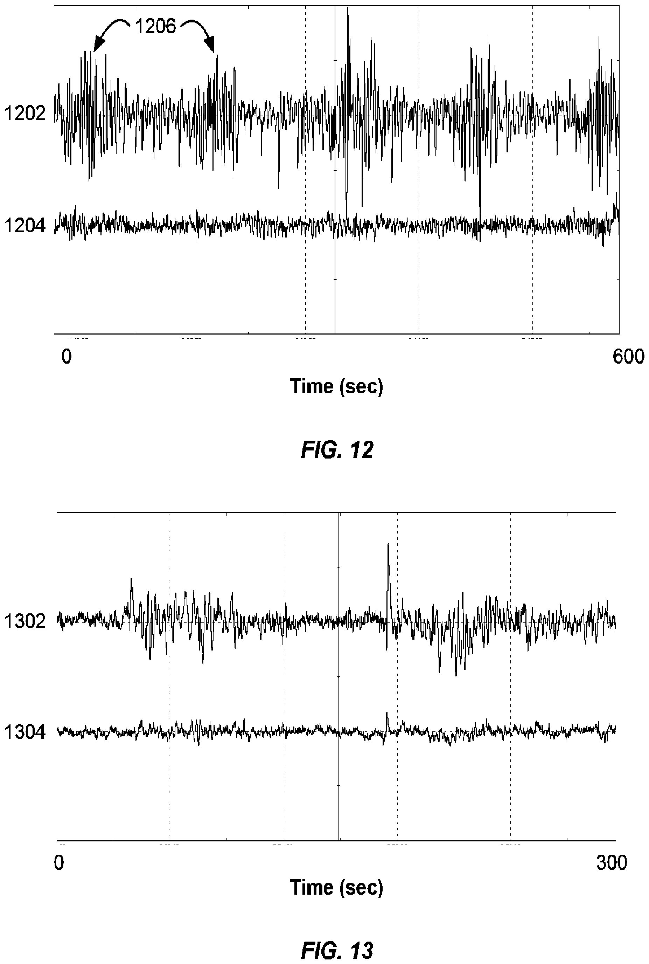

FIGS. 12 and 13 illustrative traces of a patient in active labor. To measure active labor, at least one area electrode can be placed onto a patient's abdomen in order to non-invasively assess regional contractions of the uterus.

In FIGS. 12 and 13, an area electrode is compared against other conventional techniques, such as the standard bipolar technique. The standard bipolar technique included placing two pad electrodes 5 cm apart on a patient's abdomen. An amplifier can amplify the signal(s) recorded by the electrodes in order to simultaneously display the signal voltage over time for the at least one area electrode and/or the standard bipolar pad electrodes.

FIGS. 12 and 13 illustrate assessing contractions in order to ascertain whether the patient is experiencing labor contractions. In both FIGS. 12 and 13 the top trace 1202 is the signal recorded by the area electrode, while the second trace 1204 is the signal recorded by standard bipolar electrode pads. Accordingly, the signal recorded by the standard bipolar electrode pads is barely discernable due to substantial noise interference, while the recorded signal recorded by the area electrode is clearly discernable. Specifically, in FIG. 12, the trace 1202 shows five bioelectrical bursts 1206 of about 60 second duration, each separated by about 60 to about 90 seconds, which are indicative of five consecutive uterine contractions. However, the five bioelectrical bursts were not picked up by the standard bipolar electrode pads, as can be seen in the trace 1204. These traces further demonstrate the superior signal gathering capabilities of the area electrode over the bipolar pad arrangement, but primarily serve to demonstrate that area electrode can record as many as 5 contractions over 10 minutes. Determining whether the contractions are indicative of true or active labor can be accomplished by using at least 2 and up to 20 area electrodes, thereby determining the presence or absence of synchronization of the area electrode signals. We conclude that the presence of absence of true labor can be determined using only 10 minutes of monitoring the patient.

True or active labor can be defined by a synchronization of regional contractions in the uterus (e.g., the regions are in a state of contraction during the same time period). In some aspects, there can be 20 regions (as defined by a contractile unit) of the uterus. For a patient to be in true labor, substantially all 20 regions must contract in synchrony. In other implementations, between 2 and 20, between 5 and 20, between 10 and 20, or between 15 and 20 of the regions contract in synchrony. In order to measure the synchronized contraction of these regions, at least one area electrode can be placed onto the patient's abdomen. In some aspects, the area electrode can be a 5 cm ring electrode. As discussed above, other sizes and shapes can also be contemplated.

In some aspects, the recorded signal from either one or both of the at least one area electrode and the standard bipolar pad electrodes can be filtered to reduce the SNR. This will allow any bioelectrical bursts recorded to be more visually discernable. For example, a high pass and/or a low pass filter can be implemented, where such filters may be optimal for standard bipolar techniques. A 0.3 Hz high pass and a 0.8 Hz low pass filter can be used.

The data illustrated in FIG. 13 differs from that in FIG. 12 because the patient is considered very obese, with a BMI of 42 (in comparison, the patient in FIG. 12 has a BMI of 32). With patients considered clinically obese, it is much harder to use the standard bipolar electrode technique to determine whether the patient is having a contraction. This is because the increased body fat impedes access to the uterus. However, area electrodes have been found to pick up signals better than standard bipolar electrode techniques, even in very obese patients. This is apparent in the trace 1302 of FIG. 13, where the signal recorded by the at least one area electrode has a diminished absolute voltage value in comparison to the signal recorded by the at least one area electrode in the first trace 1202 of FIG. 12. Yet, the SNR remains similar to that in FIG. 12 and the bioelectrical bursts indicative of contractions are still easily discernable. Contrastingly, the signal recorded by the standard bipolar electrode pads can perhaps be visually observed in the trace 1304 of FIG. 13, but would require detailed signal processing in order to determine the presence of a contraction. Therefore, the standard bipolar electrode pads, by themselves, are not able to accurately assess whether a very obese patient is contracting, or in true labor, while area electrodes are capable of determining if a very obese patient is having a labor contraction.

### EXAMPLE 4: COMPARISON OF AREA ELECTRODES TO STANDARD BIPOLAR TECHNIQUE IN ASSESSING FETAL HEART RATE

FIG. 14 illustrates detecting a fetal heart rate with the electrodes of the present disclosure. An area electrode was placed onto a patient's abdomen. An area electrode can be place over the fetal thorax, which is within the maternal abdomen, in order to measure fetal and/or maternal heart rate. The area electrode was a ring electrode, although other shaped area electrodes can be used.

In FIG. 14, a trace made with the area electrode is compared against other conventional techniques, such as the standard bipolar technique. The standard bipolar technique included placing two pad electrodes 5 cm apart on the patient's abdomen.

In some aspects, the recorded signal from either one or both of the area electrode and the standard bipolar technique can be filtered to optimize bioelectrical signals from the heart or EKG, and omit uterine bioelectrical activity. An amplifier can amplify the signal(s) recorded by the electrodes in order to simultaneously display the signal voltage over time for the at least one area electrode and/or the standard bipolar pad electrodes.

In FIG. 14, the first trace 1402 is the signal recorded by the area electrode, while the second trace 1404 is the signal recorded by the standard bipolar electrode pads. For example, in FIG. 14, an EKG can be used to record the electrical activity of both the maternal and/or the fetal heart rate, where a 5 Hz high pass and a 100 Hz low pass filter can be used. Note that this filter window (5 to 100 Hz) excludes uterine bioelectrical activity, which occurs between 0.2 and 2 Hz. Also note the very low values on the scale of the voltage axis indicate very low signal sizes. The maternal heart rate is the obvious sawtooth signal, but the small fetal heart rate 1406 is also easily seen midway between the first two maternal EKG signals, then recurring periodically at 400 millisecond intervals. Accordingly, the signal recorded by the standard bipolar electrode pads in the second trace 1404 is barely discernable due to substantial noise interference. In particular, the EKG recorded by the standard bipolar electrode pads is obscured by noise at 60 Hz. Note that to record the EKG signals, 60 Hz is passed through the filter window (5 to 100) and hence is obscuring. Since area electrodes are directional, 60 Hz noise pickup is also directional and there is a concomitant reduction of noise with area electrodes relative to pad electrodes. By measuring the time between occurrences of the fetal heart beats, the fetal heart rate, in terms of beats per minute, can be immediately and successively calculated and recorded. In this case, 400 milliseconds between heart beats corresponds to a fetal heart rate of 150 beats per minute.

It will be understood that various details of the presently disclosed patient matter may be changed without departing from the scope of the presently disclosed patient matter. Furthermore, the foregoing description is for the purpose of illustration only, and not for the purpose of limitation.

### C. ELECTROMYOGRAPHY BASED DRUG DELIVERY

The present solution discusses a system configured to evaluate the progress of labor induction by monitoring the uterine response to induction causing mechanical methods or drugs. The improved signal-to-noise ratio and directionality of signals recorded with the electrodes described herein can enable the detection of signals not detectable with other types of electrodes. The electromyography (EMG) signals originating from the uterus that are detected by the system described herein can be used to classify the current physiological status of the uterus to make diagnostic decisions about the infusion of induction drugs or the application of induction methods. The system can provide guidance or automate the infusion of induction drugs to quickly reach an optimum drug infusion rate. This can reduce the total time of labor, which can improve patient and fetal safety and decrease medical costs.

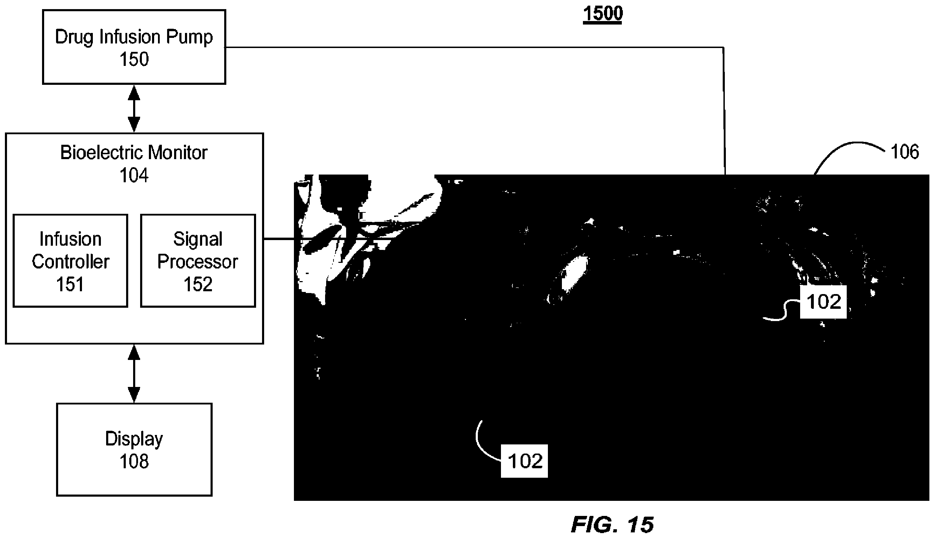

FIG. 15 illustrates an example EMG based drug delivery system 1500. The EMG based drug delivery system 1500 can be similar to the system 100 described above. For example, the EMG based drug delivery system 1500 can include a plurality of area electrodes 102 placed across the abdomen of the patient 106. The EMG based drug delivery system 1500 can also include a bioelectric monitor 104 that receives the EMG signals from each of the area electrodes 102. The bioelectric monitor 104 can display outputs, such as EMG characteristics and diagnoses, via the display 108. The bioelectric monitor 104 can control a drug infusion pump 150 that delivers drugs to the patient 106. The bioelectric monitor 104 can include an infusion controller 151 and a signal processor 152. In some implementations, the bioelectric monitor 104 can control induction methods or provide, via the display 108, recommendations for increasing, decreasing, or maintaining the mechanical induction methods.

The drug infusion pump 150 can be an intravenous (IV) drug infusion pump. The infusion controller 151 and/or the signal processor 152 can be components of the infusion pump 150 and or the bioelectric monitor 104. The infusion controller 151 can output control signals that are received by the drug infusion pump 150 to control the infusion rate of the pump.

The drug infusion pump 150 can infuse saline and other hydrating solutions into the patient 106. The drug infusion pump 150 can also infuse one or more drugs into the patient 106. The drugs can be introduced through a second infusion line that piggybacks onto the primary line containing the hydrating solutions. The drug infusion pump 150 can infuse oxytocin, other labor inducing drugs, or any other drug or fluid into the patient. The rate of the infusion can be controlled by the bioelectric monitor 104 (or an instance of the infusion controller 151 executed by the drug infusion pump 150). The drug infusion pump 150 can infuse the drug into the patient 106 at a rate of between about 1 mU/min and about 20 mU/min. In some implementations, the infusion rate can be higher than 20 mU/min. The drug infusion pump 150 can continuously infuse the drug into the primary IV line or can intermittently infuse the drug into the primary IV line. The infusion rate of the drug infusion pump 150 can also be manually set by a healthcare professional. In some implementations, the systems described herein can be used without a drug infusion pump 150 to monitor the status of labor or labor induced by non-chemical means.

The infusion controller 151 can be a script, file, program, application, set of instructions, or computer-executable code, that is configured to enable a device on which the infusion controller 151 is executed to control infusion rates. The infusion controller 151 can control the infusion rate of the drug infusion pump 150 based on a physiological or diagnosed state of the patient's uterus. The infusion controller 151 can include special purpose logic circuitry, e.g., an FPGA (field programmable gate array) or an ASIC (application specific integrated circuit). The infusion controller 151 can also include processor executable instructions that when executed by the processors or circuitry of the drug infusion system cause the infusion controller 151 to identify the LVO and the HV signals. The signal processor 152 can filter out noise signals, such as those near the 60 Hz range. The signal processor 152 can also be configured to filter out noise signals that can be introduced through movement artifact and other sources of noise. In some implementations, the signal processor 152 can be a component of the bioelectric monitor 104 and can perform signal conditioning on one or more of the electrical signals from the area electrodes 102 before passing filtered electrical signals to an instance of the infusion controller 151 executed by the drug infusion pump 150.

The infusion controller 151 can detect and identify low-voltage signals, such as low-voltage, oscillating (LVO) signals, and high-voltage (HV) oscillatory signals in the EMG signals received from the area electrodes 102. The bioelectric monitor 104 can detect synchronization between contractile units of the uterus.

The HV signals can include oscillatory signals that range from about 150 µV to about 1500 µV or between about 50 µV and about 1000 µV. The HV signals can be associated with normal uterine contractile activity. The LVO signals can range from about 20 µV to about 250 µV, between about 20 µV and about 200 µV, between about 20 µV and about 150 µV, or between about 20 µV and about 100 µV. The LVO signals can be substantially sinusoidal (or otherwise osculate). The LVO signals can have a peak to peak variability of between about 5 µV and about 30 µV, between about 10 µV and about 30 µV, or between about 15 µV and about 20 µV. The improved signal-to-noise ratio of the area electrodes 102 described herein can enable the detection of the LVO signals in a range less than 150 µV.

The LVO signals can have a frequency between about 0.20 Hz and about 2 Hz, between about 0.2 Hz and about 1.5 Hz, between about 0.2 Hz and about 1.2 Hz, or between about 0.3 Hz and about 0.6 Hz. In some implementations, the LVO signals are detected in patients that are administered oxytocin. The infusion controller 151 can classify some LVO signals as short LVO signals. Short LVO signals can have a duration less than a predetermined time duration. The predetermined time duration can be between about 10 seconds and about 120 seconds, between about 30 seconds and about 120 seconds, between about 60 seconds and about 120 seconds, or between about 60 seconds and about 90 seconds. The infusion controller 151 can classify the LVO signals as localized when the LVO signal is present in only 1, 2, or 3 of the signals from the area electrodes 102. The infusion controller 151 can classify the LVO signals as generalized when the infusion controller 151 detects the LVO signals in multiple signals from the area electrodes 102.

In some implementations, the infusion controller 151 can detect the LVO signals by low-pass filtering the signals from the area electrodes 102. For example, frequencies below about 0.5, 0.75, 1, 1.5, 3, 5, or 10 Hz can be passed through the low-pass signal. A threshold can be applied to the filtered signal to detected if LVO signals are present. For example, a threshold can be set at 50 µV. When the infusion controller 151 detects a crossing of the threshold of 50 µV, the infusion controller 151 can set a start marker indicating the start of the LVO signal. The infusion controller 151 can set an end marker indicating the end of the LVO signal once the filtered signal falls below the threshold for a predetermined amount of time. The infusion controller 151 can calculate the length of the LVO signal by subtracting the start marker time from the end marker time. In some implementations, the LVO signals can be detected by binning the signal from the area electrodes 102 into segments. A wavelet transform can be performed on each of the segments to determine if the segment includes a signal with the frequency and amplitude components of a LVO signal. Segments with LVO signal characteristics can be flagged as included an LVO signal. The length of the LVO signal can be calculated by counting the number of continuous segments identified as including an LVO signal. The infusion controller 151 can also use other frequency based analysis techniques, such as fast Fourier transforms to determine the frequency characteristics of the signals from the area electrodes 102. For example, the infusion controller 151 can use fast Fourier transforms to determine where the frequency characteristics of a LVO signal are present in the EMG signal and the HV signals can be detected by the threshold crossings.

In some implementations, the infusion controller 151 can determine the subset of the EMG channels that include LVO signals. In some cases, the infusion controller 151 may not make a diagnosis until the LVO signals are present in a predetermined number (or percentage) of the channels. For example, the diagnosis to reduce drug infusion rates may not be made until the LVO signals are detected in more than half of the channels.

In some implementations, the infusion controller 151 can set an upper threshold (e.g., 150 µV). If the filtered signal crosses the upper threshold for a predetermined amount of time, the portion of the filtered signal that crosses the upper threshold can be discarded as a possible LVO signal. The HV signals can be detected in a similar fashion but by setting a lower threshold of 150 µV and an upper threshold of 1500 µV. The infusion controller 151 can identify rest periods in the electrical signals. The rest periods can be the portions of the electrical signals that do not include HV signals and/or contraction signals.

The bioelectric monitor 104 can be configured to make clinical diagnoses. The bioelectric monitor 104 can include any script, file, program, application, set of instructions, or computer-executable code, such as the infusion controller 151, that is configured to enable a processor of the bioelectric monitor 104 to make clinical diagnoses. Responsive to a determined diagnosis, the infusion controller 151 can control the drug infusion pump 150 to infuse more, less, or the same amount of drug into the patient. The bioelectric monitor 104 can receive electrical signals from between about 2 and about 16, between about 4 and about 14, between about 6 and about 12, or between about 7 and 9 area electrodes 102.

The clinical diagnoses can indicate the patient state as no labor, normal labor, normal induced labor, excessive stimulation, or insufficient stimulation. The bioelectric monitor 104 can determine the patient is in normal labor when the bioelectric monitor 104 detects synchronized, HV signals with substantially no LVO signals between the HV signals.

The bioelectric monitor 104 can determine the patient is in normal induced labor when the infusion controller 151 detects frequent HV signals with infrequent or occasional LVO signals. The occasional LVO signals can include the presence of LVO signals that last less than 3 minutes. When the infusion controller 151 determines the patient is in normal, induced labor, the bioelectric monitor 104 can instruct the drug infusion pump 150 to maintain the current rate of drug infusion.

The bioelectric monitor 104 can determine the patient is in excessive stimulation when the infusion controller 151 detects few HV signals and frequent LVO signals. In some implementations, the bioelectric monitor 104 determines the patient is in excessive stimulation when the infusion controller 151 detects uterine fibrillation, which can be characterized as LVO signals lasting more than 2, 3, 4, or 5 minutes. When the infusion controller 151 detects excessive stimulation, the bioelectric monitor 104 can instruct the drug infusion pump 150 to stop or reduce the infusion of drug into the patient.

The bioelectric monitor 104 can determine the patient is in insufficient stimulation when the infusion controller 151 detects few HV signals and few LVO signals. The infusion controller 151 can repeatedly fill a buffer with EMG recordings and analyze the EMG signals in the buffer for the presence of LVO or HV signals. Each full buffer can be referred to as an analysis period. The buffer can include between about 1 minute and about 30 minutes, between about 1 minute and about 20 minutes, or between about 1 minute and 10 minutes of EMG signals. In some implementations, the length of the analysis period can be set by a healthcare professional. When the infusion controller 151 detects the presence of insufficient stimulation, the infusion controller 151 can increase the rate of drug infusion into the patient.

Over an analysis period, the bioelectric monitor 104 can report EMG characteristics, such as the number of HV and LVO signals present in the EMG signals, the faction of time the HV signals and the LVO signals over the analysis period, the ratio of HV to LVO signals, the presence or absence of prolonged LVO signals (or uterine fibrillation), synchronization between contractile units, or the number of coordinated HV signals or contractions that occur. The bioelectric monitor 104 can use any of the EMG characteristics to make diagnoses. In some implementations, the bioelectric monitor 104 can also receive signals from other monitors such as an intrauterine pressure catheter or tocodynamometer. The intrauterine pressure catheter and tocodynamometer can transmit pressure signals to the bioelectric monitor 104. Peaks in the pressure signals can be indicative of the patient experiencing a contraction.

In some implementations, rather than directly controlling the drug infusion pump 150, the bioelectric monitor 104 can display the determined diagnosis on the display 108 and a healthcare professional can set the infusion rate of the drug infusion pump 150. In some implementations, the display 108 can display an alert if excessive stimulation is detected. The bioelectric monitor 104 can also generate auditory alerts in response to the detection of excessive stimulation.

The below table indicates the diagnosis (or status) the bioelectric monitor 104 assigns to the patient based on the received EMG signals. The bioelectric monitor 104 can also assign the status based on input from a secondary monitor, such as an intrauterine pressure catheter or tocodynamometer. The table also illustrates the instructions the bioelectric monitor 104 supplies to the drug infusion pump 150.

| **Status** | **Secondary Monitor Input** | **Detected EMG Characteristics** | **Instructions to infusion pump** |
|---|---|---|---|
| No contractions | No contractions | No HV, no LVO, no prolonged LVO | Increase infusion rate |
| Insufficient stimulation | Few contractions | Few synchronous HV, few LVO, no prolonged LVO | Increase infusion rate |
| Optimal stimulation | 3 to 4 contractions/min | Many synchronous HV, few LVO, no prolonged LVO | Maintain infusion rate |
| Excessive stimulation | Any number of contractions | Few synchronous HV, many sync LVO, some prolonged LVO | Reduce infusion rate |
| Hyperstimulation | 5 or more contractions/min | No synchronous HV, many synchronous prolonged LVO | Stop infusion |

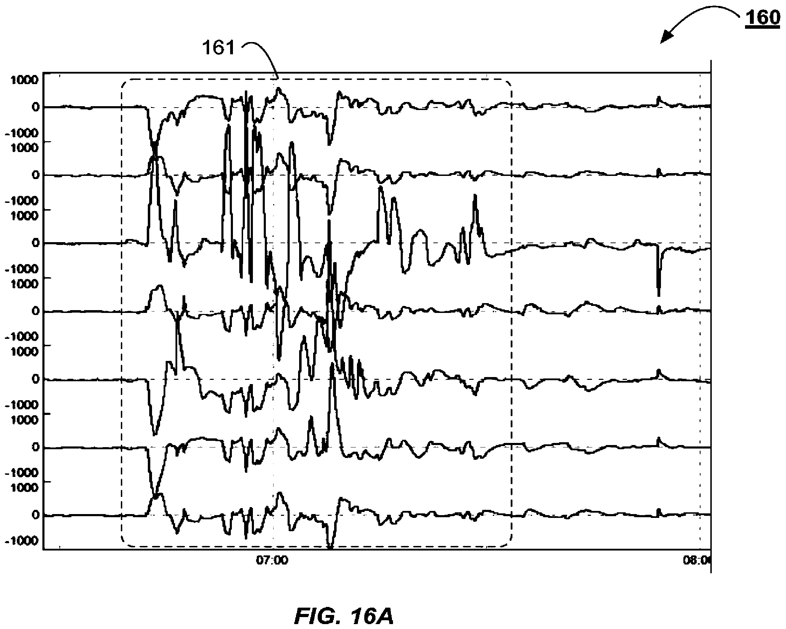

FIG. 16A illustrates a plot 160 of seven uEMG signals. Each of uEMG signals were captured with the area electrodes 102 described herein. The uEMG signals each include a portion 161 of the signal that includes HV signals. The HV signals range between about 150 µV and about 1500 µV. The HV signals are seen in each of the channels at substantially the same time. The presence of HV signals in each of the channels can indicate a strong, normal uterine contraction. The uEMG signals do not substantially contain EMG activity between neighboring uterine contractions.

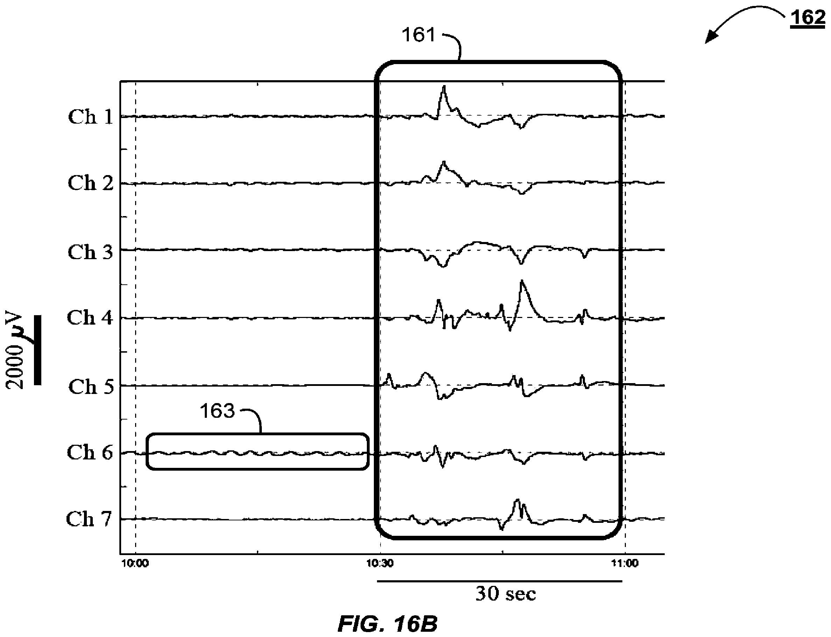

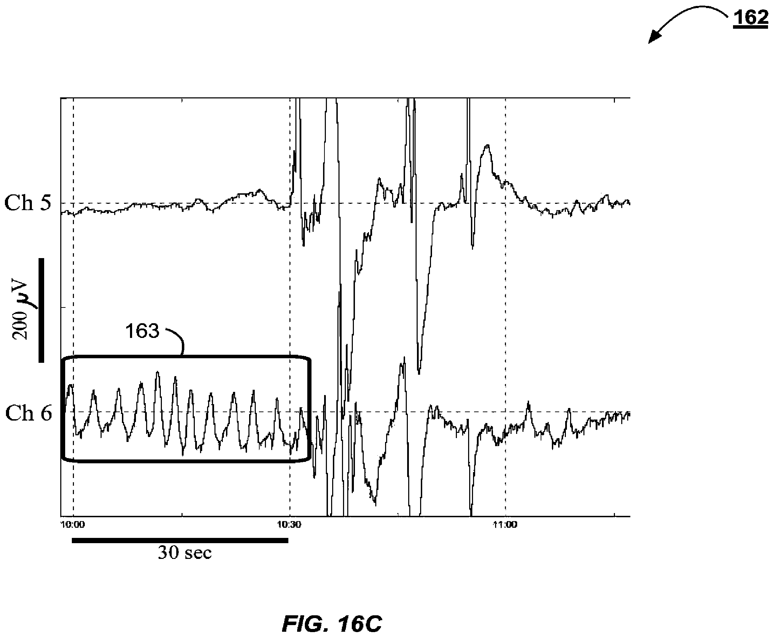

FIG. 16B illustrates a plot 162 of seven uEMG signals. Each of uEMG signals were captured with the area electrodes 102 described herein. The uEMG signals each include a portion 161 of the signal that includes HV signals. As illustrated, the HV signals are not necessarily pseudo-sinusoidal (e.g., the HV signals are not repetitive from contraction to contraction). FIG. 16B illustrated that the HV signal in each of the channels (and from each region) is unique in appearance. The peak to peak voltage changes are between 50 and 1000 µV, when filtered between 0.2 and 1.2 Hz. The HV signals can be associated with contractions of both false and true labor. The plot 162 also illustrates a portion 163 of the signal that includes a LVO signal prior to the HV signal. FIG. 16C illustrates an enlarged view of the plot 162 around the portion 163 of the signal that includes the LVO signal. FIG. 16C illustrates that the LVO signals can include regular, nearly sinusoidal components with consistent peak-peak amplitudes. In this example, the LVO is localized and is not present in the adjacent sensor (channel 5).

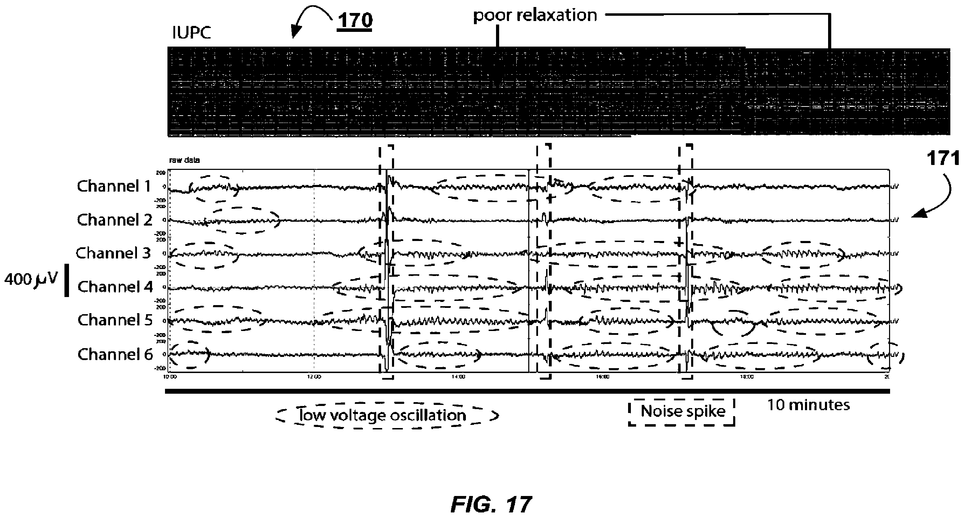

FIG. 17 illustrates a plot 170 of intrauterine pressure measured using an intrauterine pressure catheter (IUPC) and a plot 171 including six uEMG channels recorded with the area electrodes 102 described herein. The measurements were made while the patient was undergoing oxytocin administration. As illustrated in the IUPC plot 170, over the course of about 10 minutes the patient experienced 5 contractions, labeled 1-5 in the IUPC plot 170. The plot 171 illustrates the presence of LVO signals (as indicated by the oval shaped, dashed enclosures). The plot 171 illustrates that LVO oscillations reflect that uterine activity can be responsible for the poor relaxation that occurs between contractions. For example, poor relaxation where one contraction quickly leads to another is identified between contractions 2 and 3 and contractions 4 and 5. Between these contractions, LVO signals are detected in most of the uEMG channels. In contrast to the uEMG signals illustrate in FIG. 16, the HV signals associated with normal uterine contractions are absent. The bioelectric monitor 104 can be configured to identify the uEMG activity illustrated in FIG. 17 as abnormal uterine activity and classify the signals as an excessive stimulation diagnosis.

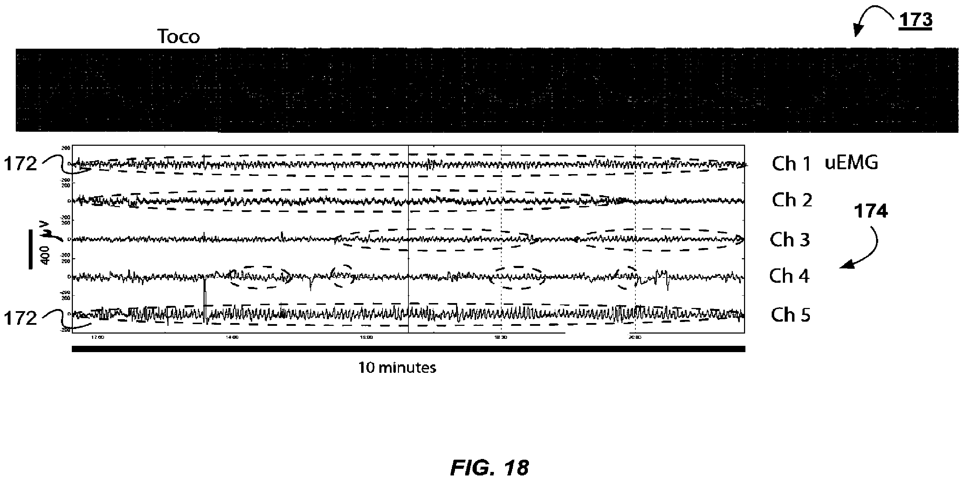

FIG. 18 illustrates a plot 173 ofcontractions measured using a tocodynamometer (toco) and a plot 174 including five uEMG channels recorded with the area electrodes 102 described herein. The plot 173 illustrates an example instance of uterine tachysytole, which is clinically defined as 6 or more contractions in 10 minutes. The plot 174 includes two channels with prolonged LVO signals 172. In some implementations, a prolonged LVO signal 172 is a LVO signal that lasts for more than 3 minutes. The prolonged LVO signal 172 can be referred to as a uterine fibrillation. The plots 173 and 172 illustrates that there is incomplete uterine rest between contractions (especially between contractions 3 and 4 and between contractions 5 and 6). The LVO signals in channels 1, 4, and 5 span nearly the full duration of the 10 minute tracing shown (e.g., plot 174). Plot 174 illustrates an example where there are prolonged LVO signals in a subset of the channels that is more than half of the total number of channels.

In summary of FIGS. 16A-18, contractions of normal labor can include uEMG recordings having synchronized HV bioelectrical signals. Abnormal uterine activity (which can lead to an excessive stimulation diagnosis) can include excessive LVO oscillatory bioelectrical signals while normal, induced labor can include intermittent LVO signals.

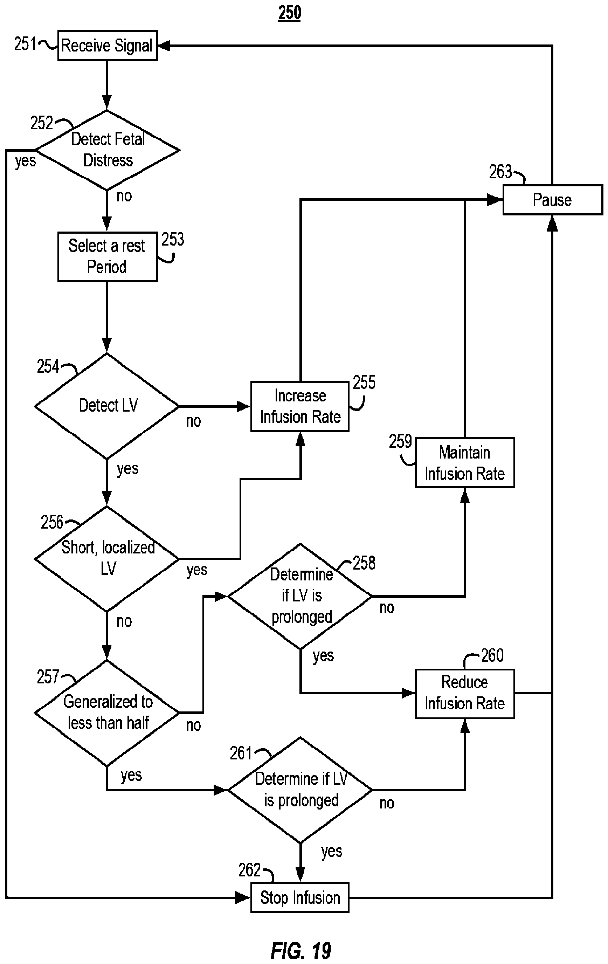

FIG. 19 illustrates a flow diagram of an example method 250 to control drug infusion. The method 250 can be performed by the infusion controller, which can be a component of the drug infusion pump 150 or the bioelectric monitor 104. The method 250 can begin with the infusion controller receiving a plurality of electrical signals (step 251). The electrical signals can be received from area electrodes that are monitoring the localized, bioelectrical signals from different regions of a uterus. The area electrodes can be similar to those described above in Sections A and B. The infusion controller can receive between about 2 and about 12, between about 4 and about 8, or between about 4 and about 6 electrical signals from a plurality of the area electrodes. The drug infusion pump 150 or the bioelectric monitor 104 that receives the electrical signals can filter the signals. The filtering can include band-pass or low-pass filtering the signals to remove the relatively high frequency components of the electrical signals. The signal processor can filter the electrical signals to pass the frequency components between about 0 Hz and about 3 Hz, between about 0.2 Hz and about 2 Hz, between about 0.2 Hz and about 1.5 Hz, between about 0.2 Hz and about 1.2 Hz, or between about 0.3 Hz and about 0.6 Hz. In some implementations, the infusion controller can perform the method 250 on a moving window of the electrical signals. For example, every 10, 30, 60, 90, or 120 seconds, the infusion controller can analyze the past 30 seconds, 1 minute, 5 minutes, 10 minutes, or 20 minutes of the electrical signal. In some implementations, the moving windows can overlap and in other implementations the windows may not overlap. For example, in a non-overlapping example, every 5 minutes, the infusion controller can analyze the past 5 minutes of data. In an overlapping example, every 30 seconds the infusion controller can analyze the past 10 minutes of data.

The method 250 can include detecting fetal distress (step 252). In some implementations, the infusion controller can continuously monitor the electrical signals or other inputs for signals of fetal distress outside the other steps of the method 250. For example, the detection of fetal distress can act as an interrupt on the method 250 and stop the method 250 and the infusion of drug if fetal distress is detected. The infusion controller can determine the presence of fetal distress based on fetal heart rate, fetal heart rate variability, fetal heart rate accelerations, or fetal heart rate decelerations. For example, normal fetal heart rate can be between about 115 and about 140 beats/min. The infusion controller can indicate fetal distress when the heart rate deviates from the normal fetal heart rate. The infusion controller can indicate fetal distress when the fetal heart rate briefly rises between about 15 and about 30 beats/min (e.g., during times of fetal heart rate acceleration), when the fetal heart rate briefly decreases below a baseline (e.g., during times of fetal heart rate deceleration), when the fetal heart rate varies during a given window, when fetal heart rate decelerates late with respect to a contraction, or other special pattern are detected in the fetal heart rate or EKG.

If fetal distress is not detected, the method 250 can include determining the presence of a rest period (step 253). The infusion controller can determine the presence of a rest period based on the electrical signals received at step 251 and/or other diagnostic inputs. For example, the other diagnostic inputs can include signals from a tocodynamometer. The infusion controller can select the portions of the electrical signal that do not include contraction and/or HV signals. The infusion controller can select the rest period as the portions of the electrical signals between the end of a first contraction and the beginning of a second contraction. In some implementations, the infusion controller can automatically determine the beginning and end of a contraction. In some implementations, the infusion controller can receive a signal that indicates the start and end of a contraction. For example, the infusion controller can mark the beginning of a contraction when the tocodynamometer signal crosses above a predetermined threshold and can mark the end of a contraction when the tocodynamometer signal falls below a predetermined threshold. The infusion controller can also mark the beginning and the end of the contractions based on the components of the electrical signals received from the plurality of area electrodes. For example, the infusion controller can determine a contraction is occurring when the amplitude of HV signals cross a predetermined threshold. The infusion controller can also select portions of the electrical signals that do not include HV signals as rest periods. The HV signals can have peak to peak amplitudes between about 50 and about 100 µV in a signal that is band-pass filtered between about 0.2 Hz and about 1.2 Hz. In some implementations, if the infusion controller cannot identify a rest period in the portion of the electrical signals being analyzed, the infusion controller can reduce or stop the drug infusion. In some implementations, the infusion controller can reduce or stop the drug infusion if the duration of the rest period is less than about 3 minutes, 2 minutes, or 1 minute.

The method 250 can include detecting LVO signals (step 254). The infusion controller can monitor each of the plurality of received electrical signals for LVO signals. In some implementations, the infusion controller can monitor a filtered version of the electrical signals. The signal processor can generate filtered electrical signals by filtering the electrical signals to pass the frequency components between about 0 Hz and about 3 Hz, between about 0.2 Hz and about 2 Hz, between about 0.2 Hz and about 1.5 Hz, between about 0.2 Hz and about 1.2 Hz, or between about 0.3 Hz and about 0.6 Hz. The infusion controller can set a bit flag for each of the electrical signals in which the infusion controller detects a LVO signal. For example, if the infusion controller receives an electrical signal from six area electrodes, the infusion controller can create a six bit array and set the bit corresponding a respective area electrode to one when the infusion controller determines the electrical signal from the respective area electrode includes a LVO signal.

If, at step 254, the infusion controller does not detect LVO signals, the infusion controller can generate an output signal that causes the drug infusion rate to increase (step 255). In some implementations, the infusion controller can also generate a notification signal. The infusion controller can transmit the notification signal to a display for presentation to a user. In some implementations, the display can be a monitor that provides text-base, image-based, or other forms of notification to the user that the infusion controller is increasing the infusion rate. In some implementations, the notification signal can be rendered by a speaker. For example, the notification signal can cause a speaker to produce an auditory alarm or notification. In some implementations, the display can be a plurality of color coded light emitting diodes (LEDs). The notification signal can cause one or more of the LEDs to illuminate. For example, when the infusion controller is increasing the drug infusion rate, the infusion controller can generate a notification signal that causes a green LED to blink.

The method 250 can include determining if the LVO signals are short and localized (step 256). If the LVO signals are short and/or localized, the infusion controller can increase the drug infusion rate (step 255). To determine whether the LVO signals are localized, the infusion controller can determine which subset of the electrical signals include a LVO signal. For example, infusion controller can set the subset of the electrical signals that include a LVO signal as the electrical signals that have a bit flag set in the above-described bit array. The infusion controller can sum the values of the bit array to determine the number of electrical signals in the subset that include a LVO signal. The infusion controller can determine that the LVO signals are localized when the number of electrical signals in the subset is below a predetermined threshold. For example, the infusion controller can determine the LVO signals are localized when the subset only includes 1, 2, or 3 electrical signals. In some implementations, the LVO signals are localized when a LVO signal is detected in only a single electrical signal.

The method 250 can include determining if the LVO signals are generalized to less than half of the electrical signals after determining that the LVO signals are not short or localized (step 257). In some implementations, step 257 can include determining whether the LVO signals have generalized to 25%, 50%, 75%, or other number of the electrical signals. The infusion controller can determine whether the LVO signals have generalized to more than or less than the threshold level by counting the number of electrical signals that include a LVO signal. When the threshold is half of the electrical signals, the infusion controller can determine that the LVO signals have not generalized to half of the electrical signals if fewer than half of the electrical signals include a LVO signal.

If at step 257, the infusion controller determines the LVO signals have not generalized, the method 250 can include determining if the LVO signals are prolonged (step 258). For each of the electrical signals that include a LVO signal, the infusion controller can determine a duration of the LVO signal. The infusion controller can set the duration for the LVO signal to the length of time that the respective electrical signal includes substantially continuous characteristics that match those of a LVO signal. For example, the infusion controller can set the duration of a LVO signal as the time between the beginning of a low voltage (e.g., 10 µV to 200 µV) oscillatory signal and the end of the low voltage oscillatory signal. The infusion controller can include a time threshold. The infusion controller can set LVO signals with a duration above the time threshold as being prolonged. In some implementations, the time threshold is between about 10 seconds and about 120 seconds, between about 20 seconds and about 120 seconds, between about 30 seconds and about 120 seconds, between about 60 seconds and about 120 seconds, or between about 60 seconds and about 90 seconds.

If at step 258, the LVO signals are not prolonged, the method 250 can include maintaining the infusion rate (step 259). Maintaining the infusion rate can include not modifying or altering the current settings of the drug infusion rate. The step 259 can include the infusion controller generating a notification signal that is transmitted to the display for rendering to the user. The notification signal can activate a green LED that indicates to the user that the infusion rate is being held constant.

If at step 258, the LVO signals are prolonged, the method 250 can include reducing the drug infusion rate (step 260). The infusion controller can reduce the drug infusion rate by between about 5% and about 75%, between about 10% and about 75%, between about 25% and about 75%, or between about 50% and about 75%. In some implementations, during the step 260, the infusion controller can generate a notification signal that is transmitted to the display for rendering to the user. The notification signal can activate a yellow LED that indicates to the user that the infusion rate is being reduced.

Returning to step 257, if the infusion controller determines the LVO signals have generalized to more than half of the electrical signals, the infusion controller can determine if the LVO signals are prolonged (step 261). As described above, the infusion controller can determine whether the LVO signals are prolonged by determining whether the duration of the LVO signals is greater than the time threshold. If, at step 261, the LVO signals are not prolonged, the infusion controller can reduce the infusion rate (step 260). If the infusion controller determines the LVO signals are prolonged, the infusion controller can stop the infusion of the drug (step 262). Responsive to determining the LVO signals are prolonged, the infusion controller can also generate a notification signal. The notification signal can activate a red LED or generate a warning message that the drug infusion rate was too high and is being stopped.

After completing steps 255, 259, 260, and/or 262, the method 250 can include pausing a predetermined amount of time (step 263). During the pause, the infusion controller can continue to monitor the input signals for fetal distress; however, the infusion controller may not generate updated instructions that cause the drug infusion rate to change. In some implementations, the length of the pause at step 263 can be different based on from which step the method 250 arrived at step 263. For example, if the infusion controller arrived at the step 263 from the step 255, the pause can be relatively short to the infusion controller can quickly recheck the electrical signals to determine whether the increased drug infusion rate is too great. If, for example, the infusion controller arrived at the step 263 form the step 262, the pause can be relatively longer. For example, the pause can be between about 20 minutes and about 30 minutes.

### D. CONCLUSION

While the following terms are believed to be well understood by one of ordinary skill in the art, the following definitions are set forth to facilitate explanation of the presently disclosed patient matter.

Following long-standing patent law convention, the terms "a", "an", and "the" refer to "one or more" when used in this application, including the claims.

Unless otherwise indicated, all numbers expressing measurements, conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed patient matter.

As used herein, the term "about", when referring to a value or to an amount of a length, width, mass, weight, temperature, time, volume, concentration, percentage, etc., is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed methods or employ the disclosed compositions.

The term "comprising", which is synonymous with "including", "containing", or "characterized by" is inclusive or open-ended and does not exclude additional, unrecited elements or method steps. "Comprising" is a term of art used in claim language which means that the named elements are essential, but other elements can be added and still form a construct within the scope of the claim.

As used herein, the phrase "consisting of" excludes any element, step, or ingredient not specified in the claim. When the phrase "consists of" appears in a clause of the body of a claim, rather than immediately following the preamble, it limits only the element set forth in that clause; other elements are not excluded from the claim as a whole.

As used herein, the phrase "consisting essentially of" limits the scope of a claim to the specified materials or steps, plus those that do not materially affect the basic and novel characteristic(s) of the claimed patient matter.

With respect to the terms "comprising", "consisting of", and "consisting essentially of", where one of these three terms are used herein, the presently disclosed and claimed patient matter can include the use of either of the other two terms.

As used herein, the term "and/or", when used in the context of a listing of entities, refers to the entities being present singly or in combination. Thus, for example, the phrase "A, B, C, and/or D" includes A, B, C, and D individually, but also includes any and all combinations and subcombinations of A, B, C, and D.

## Claims

1. A drug infusion system comprising a memory device and at least one processor executing an infusion controller configured to:
receive a plurality of electrical signals, wherein each of the plurality of electrical signals are received from a respective area electrode detecting localized bioelectrical signal from a uterus;
determine the presence of a first rest period, wherein the first rest period does not contain a uterine contraction; **characterized in that** the controller is further configured to:
identify, within the first rest period of the plurality of electrical signals, an oscillatory signal in at least one of the plurality of electrical signals, wherein the oscillatory signal has an amplitude of between about 20 µV and about 200 µV;
determine a duration of the oscillatory signal identified within the first rest period; and
reduce, responsive to determining that the duration of the oscillatory signal exceeds a threshold, an infusion rate of a drug.

2. The system of claim 1, wherein the infusion controller is further configured to:
determine, within a second rest period of the plurality of electrical signals, that the plurality of electrical signals do not include an oscillatory signal; and
increase the infusion rate of the drug.

3. The system of claim 1, wherein the infusion controller is further configured to:
identify, within a second rest period of the plurality of electrical signals, the oscillatory signal in a subset of the plurality of electrical signals; and
increase the infusion rate of the drug based on the subset of the plurality of electrical signals being below a predetermined threshold.

4. The system of claim 3, wherein the infusion controller is further configured to:
determine a duration of the oscillatory signal in the subset of the plurality of electrical signals; and
increase the infusion rate of the drug based on the duration of the oscillatory signal in the subset of the plurality of electrical signals being below a predetermined time threshold.

5. The system of claim 1, wherein the infusion controller is further configured to:
identify, within a second rest period of the plurality of electrical signals, the oscillatory signal in a subset of the plurality of electrical signals; and
decrease the infusion rate of the drug based on the subset of the plurality of electrical signals being above a predetermined threshold.

6. The system of claim 1, wherein the infusion controller is further configured to:
identify, within a second rest period of the plurality of electrical signals, the oscillatory signal is present in less than half of the plurality of electrical signals; and
maintain the infusion rate of the drug based on the oscillatory signal being present is less than half of the plurality of electrical signals.

7. The system of claim 5, wherein the infusion controller is further configured to:
determine a duration of the oscillatory signal in the subset of the plurality of electrical signals; and
stop the infusion of the drug based on the duration of the oscillatory signal in the subset of the plurality of electrical signals being above a predetermined time threshold.

8. The system of claim 7, wherein the predetermined time threshold is between about 60 seconds and about 120 seconds.

9. The system of claim 1, further comprising a signal processor configured to band-pass filter the plurality of electrical signals between about 0.2 Hz and about 2 Hz.

10. The system of claim 1, further comprising:
a signal processor configured to generate a plurality of filtered electrical signals band-pass filtered between about 0.2 Hz and about 2 Hz; and
the infusion controller further configured to determine the presence of the first rest period based on the plurality of filtered electrical signals not having peak to peak oscillations greater than 200 µV.

## Patentansprüche

1. Arzneimittelinfusionssystem, umfassend eine Speichervorrichtung und mindestens einen Prozessor, der eine Infusionssteuerung ausführt, die zu Folgendem konfiguriert ist:
Empfangen einer Vielzahl von elektrischen Signalen, wobei jedes der Vielzahl von elektrischen Signalen von einer jeweiligen Bereichselektrode empfangen wird, die ein lokalisiertes bioelektrisches Signal von einem Uterus erfasst;
Bestimmen des Vorhandenseins einer ersten Ruheperiode, wobei die erste Ruheperiode keine Uteruskontraktion enthält;
**dadurch gekennzeichnet, dass** die Steuerung ferner zu Folgendem konfiguriert ist:
Identifizieren eines oszillierenden Signals innerhalb der ersten Ruheperiode der Vielzahl von elektrischen Signalen in mindestens einem der Vielzahl von elektrischen Signalen, wobei das oszillierende Signal eine Amplitude zwischen ungefähr 20 µV und ungefähr 200 µV aufweist;
Bestimmen einer Dauer des oszillierenden Signals, das innerhalb der ersten Ruheperiode identifiziert wurde; und
als Reaktion auf das Bestimmen, dass die Dauer des oszillierenden Signals einen Schwellenwert überschreitet, Reduzieren einer Infusionsrate eines Arzneimittels.

2. System nach Anspruch 1, wobei die Infusionssteuerung ferner zu Folgendem konfiguriert ist:
Bestimmen, innerhalb einer zweiten Ruheperiode der Vielzahl von elektrischen Signalen, dass die Vielzahl von elektrischen Signalen kein oszillierendes Signal einschließt; und
Erhöhen der Infusionsrate des Arzneimittels.

3. System nach Anspruch 1, wobei die Infusionssteuerung ferner zu Folgendem konfiguriert ist:
Identifizieren des oszillierenden Signals innerhalb einer zweiten Ruheperiode der Vielzahl von elektrischen Signalen in einer Teilmenge der Vielzahl von elektrischen Signalen; und
Erhöhen der Infusionsrate des Arzneimittels auf der Grundlage der Tatsache, dass die Teilmenge der Vielzahl von elektrischen Signalen unter einem vorbestimmten Schwellenwert liegt.

4. System nach Anspruch 3, wobei die Infusionssteuerung ferner zu Folgendem konfiguriert ist:
Bestimmen einer Dauer des oszillierenden Signals in der Teilmenge der Vielzahl von elektrischen Signalen; und
Erhöhen der Infusionsrate des Arzneimittels auf der Grundlage, dass die Dauer des oszillierenden Signals in der Teilmenge der Vielzahl von elektrischen Signalen unter einem vorbestimmten Zeitschwellenwert liegt.

5. System nach Anspruch 1, wobei die Infusionssteuerung ferner zu Folgendem konfiguriert ist:
Identifizieren des oszillierenden Signals innerhalb einer zweiten Ruheperiode der Vielzahl von elektrischen Signalen in einer Untergruppe der Vielzahl von elektrischen Signalen; und
Verringern der Infusionsrate des Arzneimittels auf der Grundlage der Tatsache, dass die Teilmenge der Vielzahl von elektrischen Signalen über einem vorbestimmten Schwellenwert liegt.

6. System nach Anspruch 1, wobei die Infusionssteuerung ferner zu Folgendem konfiguriert ist:
Identifizieren, innerhalb einer zweiten Ruheperiode der Vielzahl von elektrischen Signalen, dass das oszillierende Signal in weniger als der Hälfte der Vielzahl von elektrischen Signalen vorhanden ist; und
Aufrechterhalten der Infusionsrate des Arzneimittels auf der Grundlage des Vorhandenseins des oszillierenden Signals in weniger als der Hälfte der Vielzahl von elektrischen Signalen.

7. System nach Anspruch 5, wobei die Infusionssteuerung ferner zu Folgendem konfiguriert ist:
Bestimmen einer Dauer des oszillierenden Signals in der Teilmenge der Vielzahl von elektrischen Signalen; und
Stoppen der Infusion des Arzneimittels auf der Grundlage, dass die Dauer des oszillierenden Signals in der Teilmenge der Vielzahl von elektrischen Signalen über einem vorbestimmten Zeitschwellenwert liegt.

8. System nach Anspruch 7, wobei die vorbestimmte Zeitschwelle zwischen ungefähr 60 Sekunden und ungefähr 120 Sekunden liegt.

9. System nach Anspruch 1, ferner umfassend einen Signalprozessor, der so konfiguriert ist, dass er die Vielzahl von elektrischen Signalen zwischen ungefähr 0,2 Hz und ungefähr 2 Hz bandpassfiltert.

10. System nach Anspruch 1, ferner Folgendes umfassend:
einen Signalprozessor, der so konfiguriert ist, dass er eine Vielzahl von gefilterten elektrischen Signalen erzeugt, die zwischen ungefähr 0,2 Hz und ungefähr 2 Hz bandpassgefiltert sind; und
die Infusionssteuerung, die ferner so konfiguriert ist, dass sie das Vorhandensein der ersten Ruheperiode auf der Grundlage bestimmt, dass die Vielzahl von gefilterten elektrischen Signalen keine Spitze-zu-Spitze-Schwingungen von mehr als 200 µV aufweist.

## Revendications

1. Système de perfusion de médicament comprenant un dispositif de mémoire et au moins un processeur mettant en oeuvre un dispositif de commande de perfusion configuré pour :
recevoir une pluralité de signaux électriques, dans lequel chacun de la pluralité des signaux électriques sont reçus depuis une électrode de surface respective détectant un signal bioélectrique localisé d'un utérus,
déterminer la présence d'une première période de repos, dans lequel la première période de repos ne contient pas de contraction utérine,
**caractérisé en ce que** le dispositif de commande est configuré en outre pour :
identifier, pendant la première période de repos de la pluralité des signaux électriques, un signal oscillant dans au moins un de la pluralité des signaux électriques, dans lequel le signal oscillant a une amplitude d'environ 20 µV et d'environ 200 µV,
déterminer une durée du signal oscillant identifié pendant la première période de repos, et
réduire, en réponse à la détermination que la durée du signal franchit un seuil, une cadence de perfusion d'un médicament.

2. Système selon la revendication 1, dans lequel le dispositif de commande de perfusion est configuré en outre pour :
déterminer, pendant une deuxième période de repos de la pluralité des signaux électriques, que la pluralité des signaux électriques n'incluent pas un signal oscillant, et
augmenter la cadence de perfusion du médicament.

3. Système selon la revendication 1, dans lequel le dispositif de commande de perfusion est configuré en outre pour :
identifier, pendant une deuxième période de repos de la pluralité des signaux électriques, le signal oscillant dans un sous-groupe de la pluralité des signaux électriques, et
augmenter la cadence de perfusion du médicament sur la base du fait que le sous-groupe de la pluralité de signaux électriques est inférieur à un seuil prédéterminé.

4. Système selon la revendication 3, dans lequel le dispositif de commande de perfusion est configuré en outre pour :
déterminer une durée du signal oscillant dans le sous-groupe de la pluralité des signaux électriques, et
augmenter la cadence de perfusion du médicament sur la base du fait que la durée du signal oscillant dans le sous-groupe de la pluralité des signaux électriques est inférieure à un seuil de durée prédéterminé.

5. Système selon la revendication 1, dans lequel le dispositif de commande de perfusion est configuré en outre pour :
identifier, pendant une deuxième période de repos de la pluralité des signaux électriques, le signal oscillant dans un sous-groupe de la pluralité des signaux électriques, et
diminuer la cadence de perfusion du médicament sur la base du fait que le sous-groupe de la pluralité de signaux électriques est supérieur à un seuil prédéterminé.

6. Système selon la revendication 1, dans lequel le dispositif de commande de perfusion est configuré en outre pour :
identifier, pendant une deuxième période de repos de la pluralité des signaux électriques, si le signal oscillant est présent dans moins de la moitié de la pluralité des signaux électriques, et
maintenir la cadence de perfusion du médicament si le signal oscillant est présent dans moins de la moitié de la pluralité des signaux électriques.

7. Système selon la revendication 5, dans lequel le dispositif de commande de perfusion est configuré en outre pour :
déterminer une durée du signal oscillant dans le sous-groupe de la pluralité des signaux électriques, et
stopper la perfusion du médicament sur la base du fait que la durée du signal oscillant dans le sous-groupe de la pluralité des signaux électriques est supérieure à un seuil de durée prédéterminé.

8. Système selon la revendication 7, dans lequel le seuil de durée prédéterminé est d'environ 60 secondes à environ 120 secondes.

9. Système selon la revendication 1, comprenant en outre un processeur de signal configuré pour filtrer en passe-bande la pluralité des signaux électriques entre environ 0,2 Hz et environ 2 Hz.

10. Système selon la revendication 1, comprenant en outre :
un processeur de signal configuré pour générer une pluralité de signaux électriques filtrés en passe-bande entre environ 0,2 Hz et environ 2 Hz, et
le dispositif de commande de perfusion configuré en outre pour déterminer la présence de la première période de repos sur la base du fait que la pluralité des signaux électriques filtrés ne présentent pas d'oscillation crête-à-crête supérieure à 200 µV.
